(19) **Europäisches Patentamt / European Patent Office / Office européen des brevets**

(11) **EP 4 158 340 B1**

(12) **EUROPEAN PATENT SPECIFICATION**

(45) Date of publication and mention of the grant of the patent:
**19.03.2025 Bulletin 2025/12**

(21) Application number: **21729859.5**

(22) Date of filing: **27.05.2021**

(51) International Patent Classification (IPC):
**G01N 33/53** *(2006.01)*     **G01N 33/543** *(2006.01)*
**G01N 33/487** *(2006.01)*     **C12Q 1/6834** *(2018.01)*
**B82Y 15/00** *(2011.01)*

(52) Cooperative Patent Classification (CPC):
(C-Sets available)
**G01N 33/5302; G01N 33/48721; G01N 33/54346;**
**B82Y 15/00; C12Q 1/6834** (Cont.)

(86) International application number:
**PCT/EP2021/064271**

(87) International publication number:
**WO 2021/239912 (02.12.2021 Gazette 2021/48)**

(54) **ANALYTE DETECTION METHOD**

VERFAHREN ZUM NACHWEIS VON ANALYTEN

MÉTHODE DE DÉTECTION D'ANALYTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2020 GB 202007911**

(43) Date of publication of application:
**05.04.2023 Bulletin 2023/14**

(73) Proprietor: **Imperial College Innovations Limited London SW7 2AZ (GB)**

(72) Inventors:
• **IVANOV, Aleksandar, Petrov**
  **London SW7 2AZ (GB)**
• **REN, Ren**
  **London SW7 2AZ (GB)**
• **EDEL, Joshua, Benno**
  **London SW7 2AZ (GB)**

(74) Representative: **Kilburn & Strode LLP et al Lacon London 84 Theobalds Road Holborn London WC1X 8NL (GB)**

(56) References cited:
WO-A1-2017/091724     WO-A2-2007/092434
CN-A- 111 778 244      CN-A- 112 795 628
US-A1- 2019 062 814

• CARBONARO A ET AL: "A resistive-pulse sensor chip for multianalyte immunoassays", LAB ON A CHIP, ROYAL SOCIETY OF CHEMISTRY, UK, vol. 5, no. 10, 1 January 2005 (2005-01-01), pages 1155 - 1160, XP008080706, ISSN: 1473-0197, DOI: 10.1039/B504827C
• XIAOYAN LIN ET AL: "Selective single molecule nanopore sensing of proteins using DNA aptamer-functionalised gold nanoparticles", CHEMICAL SCIENCE, vol. 8, no. 5, 1 January 2017 (2017-01-01), United Kingdom, pages 3905 - 3912, XP055479944, ISSN: 2041-6520, DOI: 10.1039/C7SC00415J
• AKAHORI RENA ET AL: "Japanese Journal of Applied Physics Target DNA detection by solid-state nanopores using gold nanoparticle probes Target DNA detection by solid-state nanopores using gold nanoparticle probes", JPN. J. APPL. PHYS, 1 September 2020 (2020-09-01), pages 107001, XP055825173, Retrieved from the Internet <URL:https://iopscience.iop.org/article/10.35848/1347-4065/abb4a9/pdf> [retrieved on 20210716]

EP 4 158 340 B1

- **YAN SHAN ANG ET AL: "Rapid and Label-Free Single-Nucleotide Discrimination via an Integrative Nanoparticle-Nanopore Approach", ACS NANO, vol. 6, no. 10, 1 January 2012 (2012-01-01), US, pages 8815 - 8823, XP055557605, ISSN: 1936-0851, DOI: 10.1021/ nn302636z**
- **SALEH O A ET AL: "Direct detection of antibody-antigen binding using an on-chip artificial pore", PROCEEDINGS OF THE NATIONAL ACADEMY OF SCIENCES, NATIONAL ACADEMY OF SCIENCES, US, vol. 100, no. 3, 4 February 2003 (2003-02-04), pages 820 - 824, XP002669463, ISSN: 0027-8424, [retrieved on 20030127], DOI: 10.1073/PNAS.0337563100**
- **ESASHIKA KEIKO ET AL: "DNA Hybridization Assay Using Gold Nanoparticles and Electrophoresis Separation Provides 1 pM Sensitivity", BIOCONJUGATE CHEMISTRY, vol. 29, no. 1, 17 January 2018 (2018-01-17), US, pages 182 - 189, XP055825275, ISSN: 1043-1802, Retrieved from the Internet <URL:https://pubs. acs.org/doi/pdf/10.1021/acs. bioconjchem.7b00682> DOI: 10.1021/ acs.bioconjchem.7b00682**
- **VIVEK V. THACKER ET AL: "DNA origami based assembly of gold nanoparticle dimers for surface-enhanced Raman scattering", NATURE COMMUNICATIONS, vol. 5, no. 1, 13 March 2014 (2014-03-13), pages 1 - 7, XP055531787, DOI: 10.1038/ncomms4448**

Remarks:

The complete document including Reference Table(s) and the Sequence Listing(s) can be downloaded from the EPO website

(52) Cooperative Patent Classification (CPC): (Cont.)

C-Sets
C12Q 1/6834, C12Q 2525/207, C12Q 2563/155, C12Q 2565/631

## Description

### Field of Invention

The present invention provides a method for the detection of one or more analytes in a sample. Specifically, the invention provides a method of analyte detection involving the use of gold nanoparticles (AuNP) dimers and their detection by voltage-driven nanopore translocation.

### Background

Early and rapid diagnosis of disease is an essential step in enhancing effectiveness of treatments and in reducing morbidity and mortality. As a non-invasive and cost-effective tool for early diagnosis and prognosis of acute and chronic diseases, the detection of biomarkers, especially at a single-molecule level, have attracted researchers' attention for decades.

Meanwhile, the exquisite design of micro/nano environmental sensing methods and devices allows the detection of analytes at a single molecule level. With size-controllable features, nanopores[1, 2, 3, 4, 5] have been proven to have the capability of detecting single DNA[2, 6, 7, 8, 9], protein[10, 11, 12], nanoparticles[13, 14, 15, 16, 17] and other molecules[18, 19, 20]. The detection method is straightforward; Targeting molecules are translocated through a nanometer-sized pore using an applied electric field, and the translocations are detected by the transient changes of ionic current. The magnitude and dwell time of the current change provides information on the structural features of the analytes. However, the screening of small biomolecules using nanopore remained remarkably challenging due to their small size, heterogeneous charge, fast translocation and low signal-to-noise ratio.

Attempts have been made to address some of these limitations by functionalizing the nanopore surface with hydrophobic, and positively or negatively charged binding sites[21, 22, 23]. In addition, aptamer encoded glass nanopores[24, 25], chemically modified nanopores with binding sites[23, 26, 27], and nanopipette based field-effect-transistors[28, 29, 30] have also been investigated. However, functionalizing nanopores is often challenging due to complicated fabrication processes and the requirement for careful optimization. As another potential solution, high bandwidth amplifiers have been used to detect small molecules[31, 32]. Unfortunately, these high-resolution instruments are not capable of differentiating multiple kinds of biomolecules. Therefore, fundamental challenges remain in the sensitive and selective detection of biomarkers such as micro RNA, proteins and small antigen molecules using label-free solid state nanopores.

An alternative solution involves the use of molecular carriers, which include specific sites that interact with the molecules they transport. Singer et al. pioneered the concept of utilising the DNA as molecular carriers[33]. Bell et al. used nanopipettes and a carrier based on a 7.2 kbp DNA including a sequence of structural features (dumbbells) as a 'barcode' identifying the DNA and a binding site which can interact with targeting antibodies selectively[34]. Recently, Sze et al. designed a delicate multiple proteins carrier based on λ-DNA with ssDNA overhang which can anchor aptamers to bind targeting proteins (up to three) selectively[35 and 35a]. Combining the above concepts, more sophisticated molecular carriers have been reported[36, 37]. However, these designs are restricted by many factors such as the relatively high concentration of targeting analytes, especially proteins, and high ionic strength of buffer/sample required to prevent non-specific binding due to the shielding effect provided by salt ions. Moreover, the folding of long-chain DNA based carrier backbone[38] can cause interference in the identification of the analyte signal, while the short carriers are hard to fabricate, purify and embed the binding sites. These approaches are also not capable of sensing small molecules (<15kDa) due to the poor signal-to-noise ratios. WO2017091724A1 is directed to a target molecule modified to facilitate detection in a nanopore device, and a method of detecting such a modified target molecule using a nanopore device. Lin et al (Chem. Sci., 2017, 8, 3905) describes selective single molecule nanopore sensing of proteins using DNA aptamer-functionalised gold nanoparticles. Y. S. Ang et al (ACS Nano 2012, 6, 10, 8815-8823) describes a rapid and label-free single-nucleotide discrimination via an integrative nanoparticle-nanopore approach. US 2019/0062814 A1 describes nucleic acid nanoparticles for analyte detection. Saleh et al (PNAS 2003, 100, 3, 820-824) describes direct detection of antibody-antigen binding using an on-chip artificial pore. WO 2007/092434 A2 describes the use of resistive-pulse sensing with submicrometer pores or nanopores for the detection of the assembly of submicrometer or nanometer sized objects.

### Summary of invention

To address the limitations of the prior art, the present invention provides sensing strategies which aim to increase the sensitivity and selectivity of nanopore biosensing by increasing the size of the analyte, in order to improve the signal-to-noise ratio. The scope of the present invention is set out in the appended set of claims.

Accordingly, in a first aspect there is provided a method of detecting one or more analytes in a target sample, the method comprising:

a. providing a nanoparticle dimer adapted to bind the analyte;

b. causing the dimer to pass through a nanopore by voltage-driven translocation;

c. observing changes in the translocation current; and

d. comparing the translocation current profile of the target sample to the translocation current profile of a control sample;

wherein a change in the translocation current profile of the target sample versus the control sample indicates the presence of the analyte in the target sample; wherein the dimer comprises two nanoparticles linked by one or more nucleic acids, wherein at least one of the nucleic acids includes an aptamer specific for the analyte; and wherein the analyte is a protein.

The nanoparticle dimer may be adapted to bind the analyte in a number of different ways.

Preferably, the nanoparticles may be linked by partially complementary nucleic acids.

Preferably, the dimer comprises two nanoparticles, each of which is attached to a nucleic acid, and each of the nucleic acids includes part of an aptamer specific for the analyte, such that in the presence of the analyte an aptamer is formed and thereby a dimer is formed.

Preferably, the nanoparticles in the dimer are of substantially the same diameter. Alternatively, the nanoparticles in the dimer may be of different diameters.

Preferably, the nanopore is the tip of a nanopipette.

Preferably, the nanoparticles are gold nanoparticles (AuNPs).

Preferably, the target sample is a biological sample selected from blood, serum, lymph, sputum, urine, faeces, semen, sweat, tears, amniotic fluid, cerebrospinal fluid (CSF) and wound exudate.

The biological sample may alternatively be any other bodily fluid or secretion in a state of health or disease.

The present inventors have exemplified the first aspect of the invention using a first strategy and two other strategies are described. The Examples of the present application relate to gold nanoparticles (AuNPs), but it will be appreciated that the invention is applicable to any nanoparticle which is suitable (in terms of its size and surface charge) for electrical detection when passed through a nanopore using voltage-driven translocation.

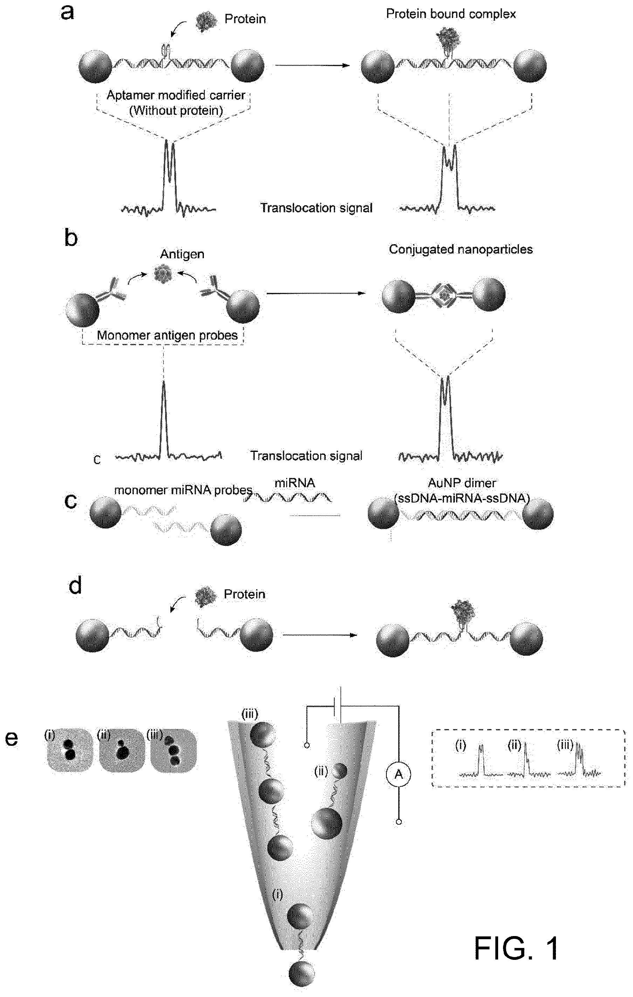

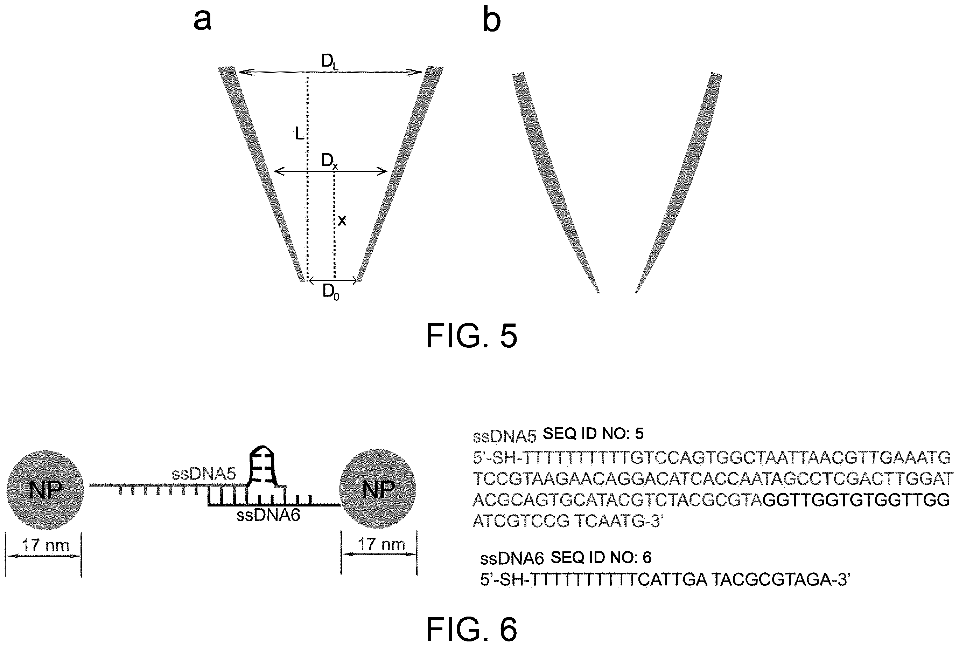

The first strategy - for transporting and identifying relatively bigger single proteins (> 35 kDa) - implemented by a carrier which is a dumbbell system comprising two nanoparticles with an aptamer located at the middle of a DNA linker (Fig. 1a). Each end of the linker is attached to one of the nanoparticles. Aptamers are ssDNA or RNA oligonucleotides that are capable of binding target molecules with high specificity and affinity, where the sequence can be selected by SELEX (systematic evolution of ligands by exponential enrichment). The specific aptamer modified dimer carriers thus bind to target proteins selectively. Then the sub-structure of protein/carrier complexes can be identified when they pass through the nanopore along with high-resolution translocation signals. A specific example of such a configuration using AuNPs and ssDNA linkers is shown in Figure 6.

A second strategy is described, which is for detecting tiny antigen molecules (< 15 kDa) and is achieved by a pair of monomer (nanoparticle) probes, one being conjugated to an antibody to a target antigen and the other being conjugated to a complementary secondary antibody (Fig. 1b). The monomer (nanoparticle) probes will self-assemble to dimer molecules by the linkage of the antibody-antigen-antibody sandwich bridge after the addition of the specific antigen. In this strategy, the method of the first aspect of the invention can also be seen to include a step of causing the nanoparticles to form a dimer.

The two strategies can be combined (Fig. 1d): one nanoparticle is functionalised with ssDNA with a part of aptamer sequence at the end while the other nanoparticle is functionalised with ssDNA that includes the rest of aptamer sequence; when the target protein is present, the two aptamer parts will link together to form an aptamer which binds to the protein, leading to the conjugation of the nanoparticle monomers. It can thus be seen that in this embodiment the dimer is formed by formation of the aptamer. In this embodiment, the method of the first aspect of the invention can also be seen to include a step of causing the nanoparticles to form a dimer.

A third strategy is described, which can be used for the selective detection of nucleic acid molecules such as miRNA (Fig. 1c). Two populations of nanoparticles can be modified to each contain half (or at least two different portions) of a sequence complementary to an miRNA sequence. In the presence of miRNA, the monomeric nanoparticles will self-assemble and dimerize.

In all strategies, the translocation signal of the nanoparticle dimer compared to individual nanoparticles indicates the presence of the analyte. This allows for very accurate detection of analytes based on the peak shape. All strategies can be adapted to different targets (including small molecules such as small peptides and proteins) by changing the aptamer sequence or antibody to the required target. In addition, these strategies can be adapted to quantify concentration of the target. The strategies also allow detection of analytes in complex media as the analytes are not detected directly but rather via the formation of dimers.

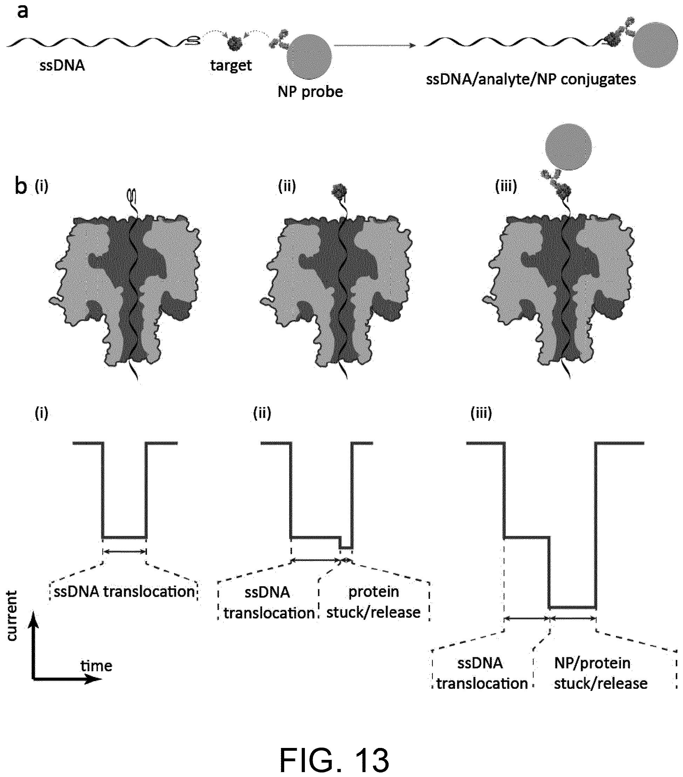

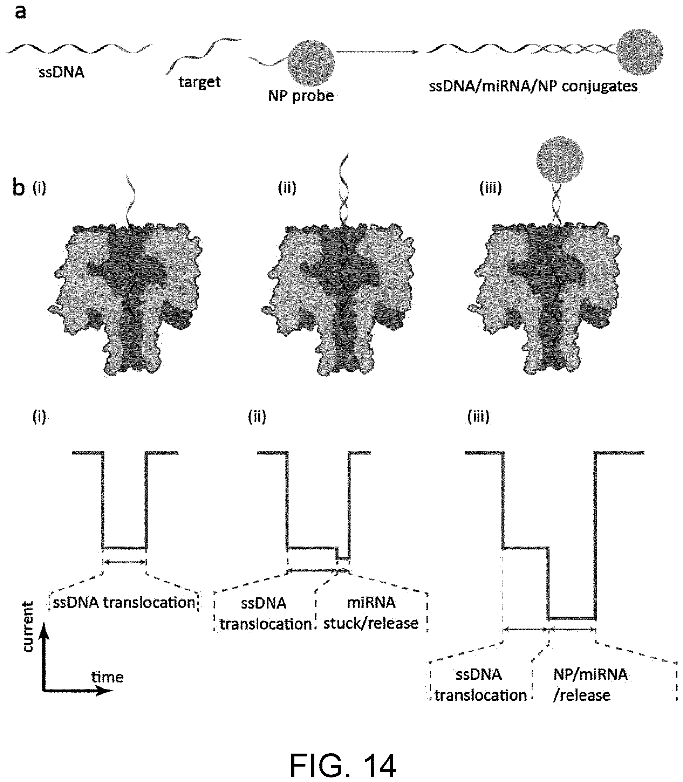

The present inventors have also devised a strategy for using nanopore monomers for sensing analytes using functionalised nanoparticle probes and DNA carriers by biological nanopores. These aspects of the invention are shown in Figures 13 and 14, which illustrate the methods for AuNP probes.

## Description of Drawings

**Figure 1.** Schematic of dimer protein carrier and monomer antigen probes.
The dimer carrier (a) is based on a dumbbell system where two nanoparticles (for example Au nanoparticles (17-20 nm)) are linked by a ~110-mer DNA bridge which is engineered to contain an aptamer sequence that binds to target protein molecules. The monomer probes (b) contain two types of nanoparticle, where each nanoparticle is modified with a different antibody, and the two nanoparticles bind together with the presence of the target antigen. Driven by the electrical field, the nanoparticle dimer carriers with target protein or the dimers with the antibody-antigen-antibody sandwich bridge translocate from inside nanopipette to outside. (c) A similar strategy can be used for the selective detection of miRNA. Two populations of NPs can be modified to each contain half of the complementary miRNA sequence. In the presence of miRNA the monomeric NPs will self-assemble and dimerize. (**d**) The two strategies shown in parts (a) and (b) can be combined: one nanoparticle is functionalised with ssDNA with a part of aptamer sequence at the end while the other nanoparticle is functionalised with ssDNA that includes the rest of aptamer sequence; when the target protein is present, the two aptamer parts will link together and bind to the protein, leading to the conjugation of the nanoparticle monomers. The changes of translocation events represent the sensing of target protein or antigens at single molecule level. Also, by counting the percentage of these signatures, the concentration of the specific target molecules can be achieved. **(e)** The schematic of screening the substructure of the nanoparticle conjugates by using a nanopipette.

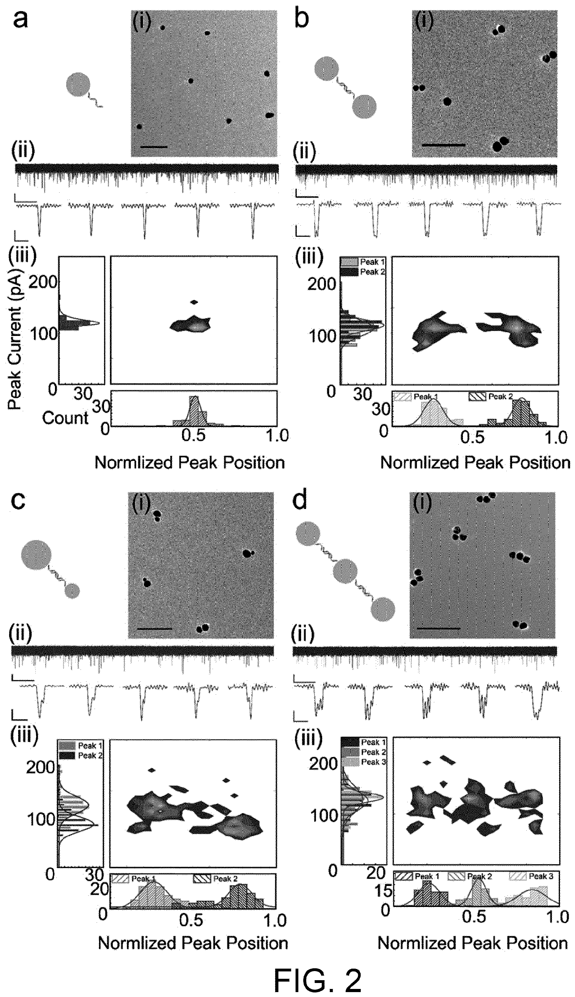

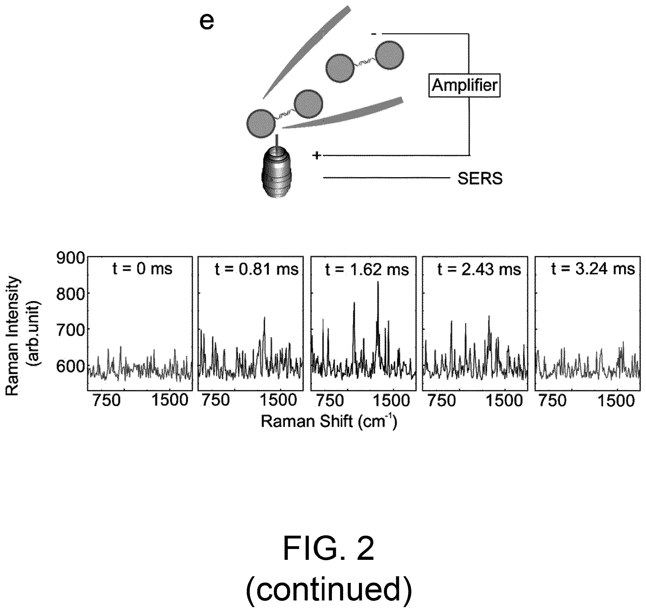

**Figure 2.** The TEM images, current-time trace, individual events, single molecule SERS and statistics of a series of AuNP based nanostructures.
The TEM (Scale bar, 100 nm), current-time trace (Scale bar: vertical 50 pA, horizontal 5 s), individual events (Scale bar: vertical 50 pA, horizontal 20 $\mu$s) and statistics for **a** AuNP monomer **b** AuNPs symmetrical dimer **c** AuNPs asymmetrical dimer and **d** AuNPs trimer. TEM images (i) indicate the clear geometry of the molecules. All the translocation experiments were performed in 50 mM KCl, 10 mM Tris-HCl, 1 mM EDTA and at a potential of -600 mV. Each current-time trace (ii) is recorded with 1 $\mu$s sampling rate and filtered using a low-pass Bessel filter with a cut-off frequency of 100 KHz. Typical individual translocation events are also shown in (ii) as they can be distinguished easily from others. Surface plots along with peak current and normalized peak position histograms are shown in (iii) clearly indicate that the translocation signal is capable of simulating the nanostructure where the size/charge of each molecule and even the distance between them. **e** PEG modified 4-ATP loaded 35 nm AuNP dimer which is capable of enhancing the Raman signal of the Raman dye in the nanogap. **f** Schematic of single molecule SERS experimental arrangement. The dimers were loaded into the gold coated nanopipette and a negative bias voltage was applied to drive the molecules to pass through the nanopore. The electrical signal and Raman signal can be recorded simultaneously. **g** Raman spectra showing the translocation of dimers. An example of 4.05 ms translocation using a - 800 mV potential at an 810 $\mu$s acquisition time. These spectra show single AuNP dimer is translocating through the nanopore over the time period of 4.05 ms.

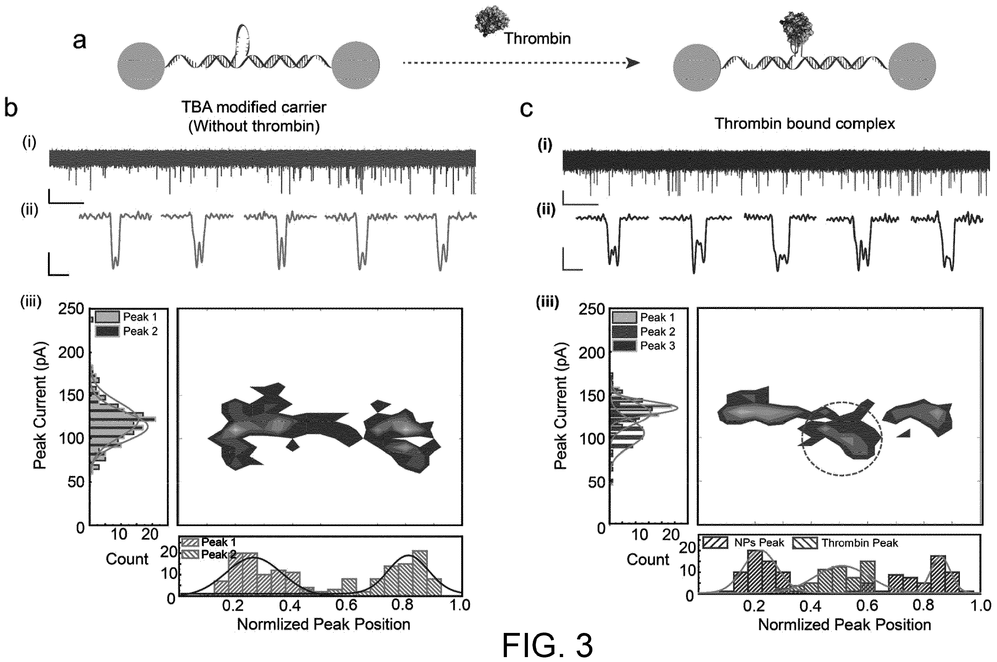

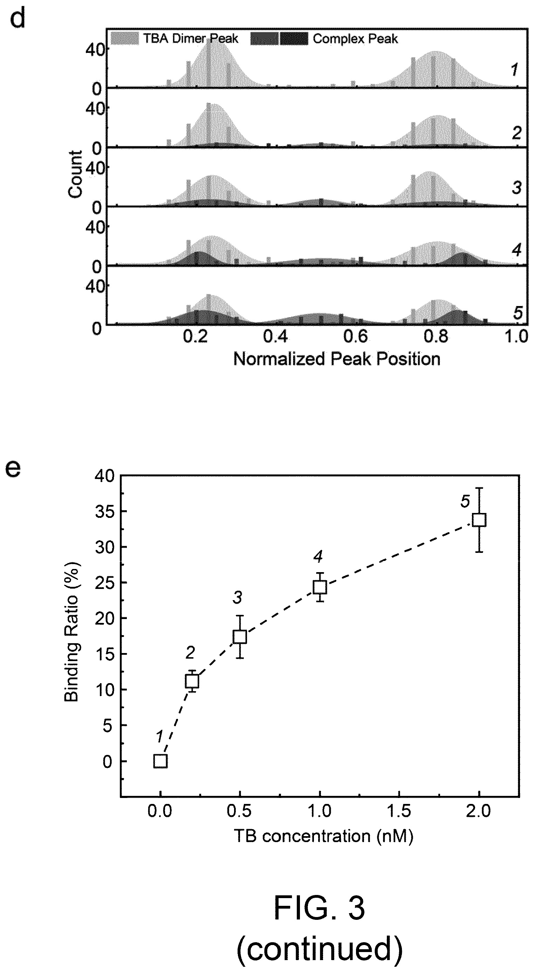

**Figure 3.** Nanopore sensing of thrombin with AuNP dimer thrombin carriers.
**a** The schematic of the dimer carriers, which engineered to contain aptamer sequences, deliver and sense the target protein. **b-c** The representative current-time trace (Scale bar: vertical 50 pA, horizontal 5 s), individual translocation events (Scale bar: vertical 50 pA, horizontal 20 $\mu$s) and statistics for AuNPs dimer thrombin carriers and AuNPs dimer with thrombin complex. The translocation experiments were performed in 50 mM KCl, 10 mM Tris-HCl, 1 mM EDTA and at a potential of -600 mV. Each trace is recorded with 1 $\mu$s sampling rate and filtered using a low-pass Bessel filter with a cut-off frequency of 100 KHz. **d** Under different concentration of thrombin (From top to bottom, 0 nM, 0.2 nM, 0.5 nM, 1 nM, and 2 nM), the normalized peak position of the dimer carriers or the carrier and target protein complexes. **e** Binding assay for a 1 nM dimer carrier concentration incubated with increasing thrombin concentration ranging from 0 to 2 nM. Error bars represent the standard deviation of three independent experimental repeats with different nanopipettes.

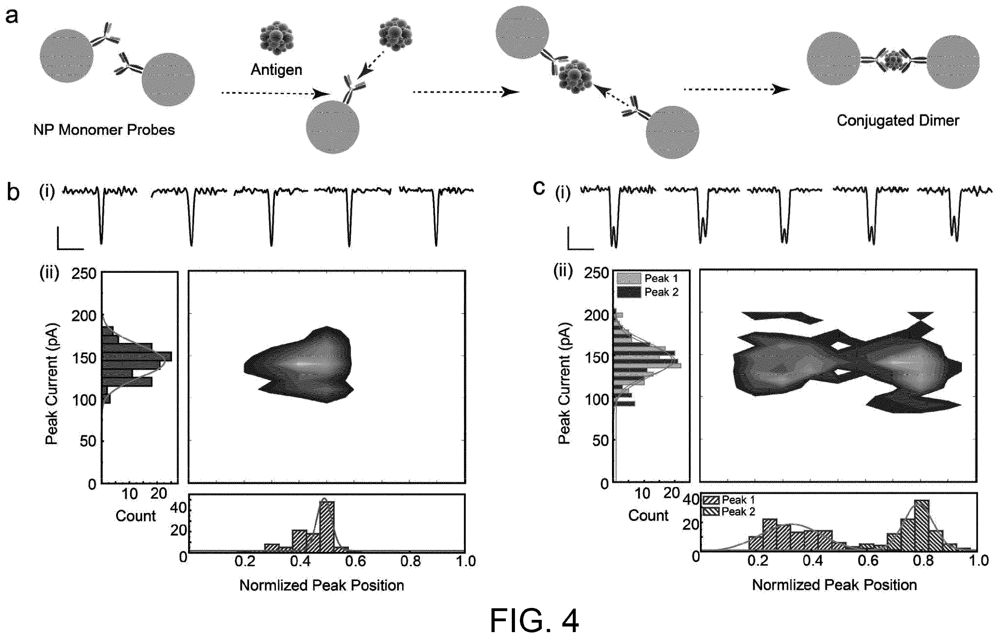

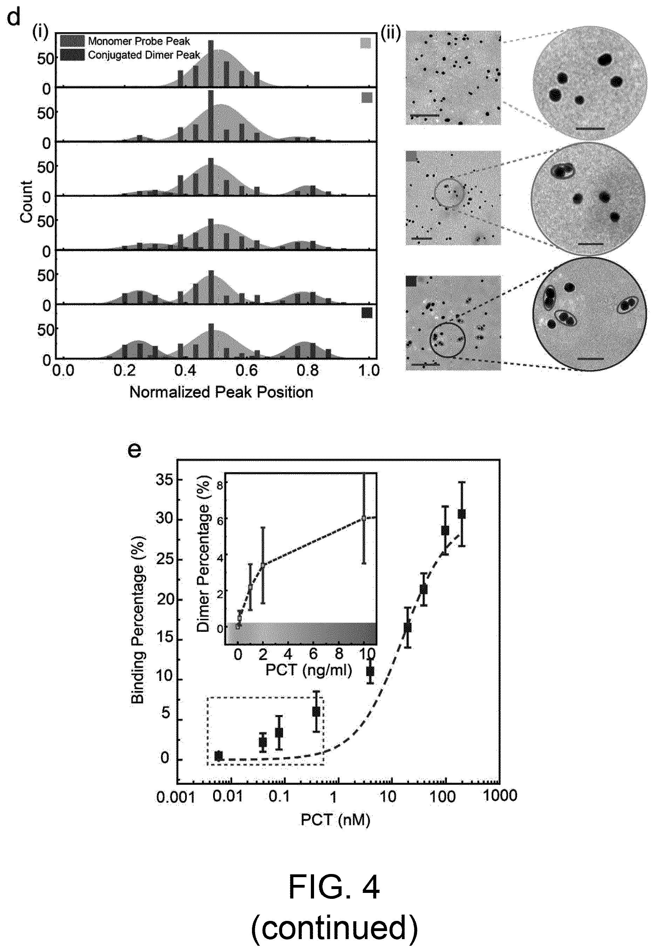

**Figure 4.** Nanopore sensing of procalcitonin (PCT) via a pair of AuNP monomer probes.
**a** The schematic of two AuNP monomer probes with different antibody will self-assemble to dimer with the presence of antigen (procalcitonin (PCT)). **b-c** The individual translocation events (Scale bar: vertical 50 pA, horizontal 20 $\mu$s) and

statistics of AuNPs monomer probes conjugated dimers which linked by antibody-antigen-antibody sandwich bridges. All the translocation experiments were performed in 50 mM KCl, 10 mM Tris-HCl, 1 mM EDTA and at a potential of -600 mV. The data was recorded with 1 μs sampling rate and filtered using a low-pass Bessel filter with a cut-off frequency of 100 KHz. **d** The normalized peak position of the monomer probe and conjugated dimer with different concentration of PCT (From top to bottom, 0 nM, 80 pM, 20 nM, 40 nM, 100 nM and 200 nM). The TEM images are used to confirm the result of binding ratio (Scale bar: 200 nm, enlarged, 50 nm). **e** the binding curve of 1 nM AuNP monomer PCT probes incubating with PCT ranging from 0 to 400 nM. The inset is the sensing of PCT in clinical diagnostic range. Error bars of binding ratio analysis represent the standard deviation of three independent experimental repeats.

**Figure 5.** Schematic representation of a nanopipette.
**a** the model of the conical nanopore for effective length calculation. b the reality nanopore shape model.

**Figure 6.** The details of AuNP dimer thrombin carrier.
The AuNP dimer thrombin carrier is self-assembled by an AuNP with a 115-mer ssDNA (ssDNA5) containing 15-mer thrombin-binding aptamer (TBA) sequence (shown in black in Figure 6) and an AuNP with the complementary part to the sequence aside of the aptamer part (ssDNA6). After the self-assembling, the protein carrier with a prominence of aptamer can sense the thrombin selectively. It is noteworthy that there are 8 random bases close to the aptamer sequence to minimize the steric hindrance.

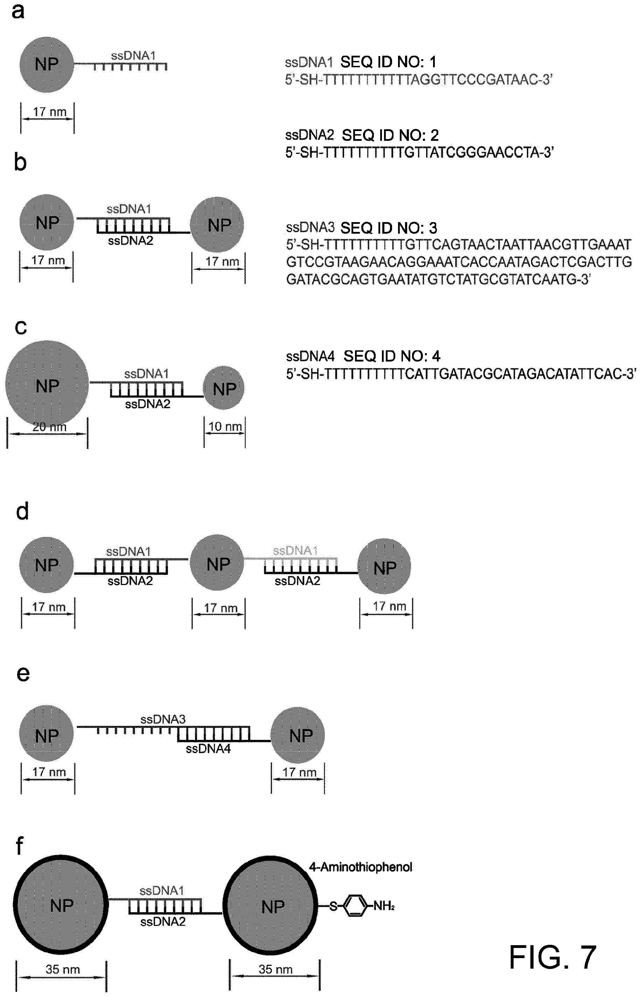

**Figure 7.** The details of AuNP based nanostructures.
**a** AuNP monomer. One 17 nm AuNP modified by a ssDNA (ssDNA1). **b** AuNPs symmetrical dimer. Two 17 nm AuNPs linked by a DNA bridge (35 bases long) (formed by hybridisation of ssDNA1 and ssDNA2). c AuNPs asymmetrical dimer. One 20 nm AuNP and one 10 nm AuNP linked by a DNA bridge (formed by hybridisation of ssDNA1 and ssDNA2) (35 bases long). **d** AuNPs trimer. Three AuNPs linked by two DNA bridges (each formed by hybridisation of ssDNA1 and ssDNA2) (each 35 bases long). **e** AuNPs symmetrical dimer with long linker. Two 17 nm AuNPs linked by a DNA bridge (formed by hybridisation of ssDNA3 and ssDNA4) (117 bases long). **f** The AuNP symmetrical dimers with Raman reporter. Two 4-aminothiophenol (4-ATP) modified (shown as black outline) 35 nm AuNPs linked by a DNA bridge (formed by hybridisation of ssDNA1 and ssDNA2) (35 bases long).

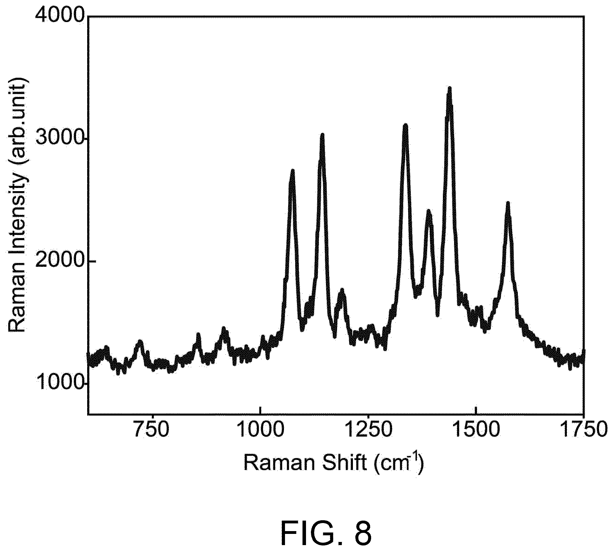

**Figure 8.** The SERS spectrum of the 4-ATP modified AuNP symmetrical dimer in bulk solution.
SERS spectrum of 4-ATP dye present in nanogap of AuNP symmetrical dimer. Spectrum of 4-ATP dye shows extremely high enhancement of the 1138, 1387, and 1571 cm$^{-1}$ vibrational bands of 4-ATP.

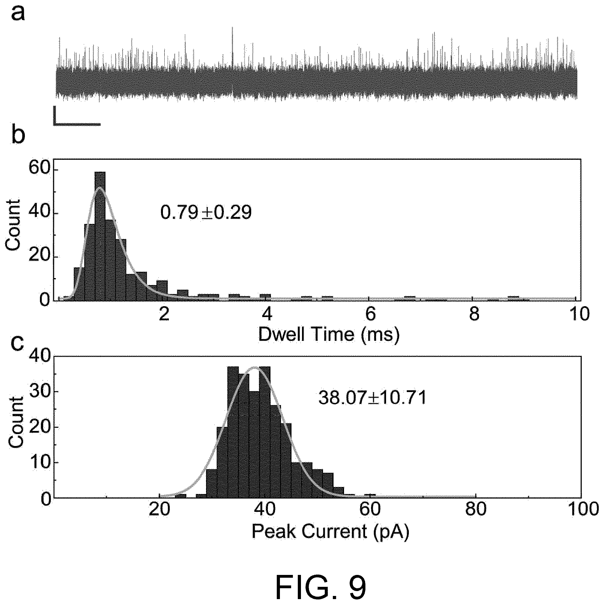

**Figure 9.** Translocation of PEG stabilized 35 nm AuNP Dimer functionalized with 4-ATP dye.
The current-time trace (Scale bar: vertical 50 pA, horizontal 5 s and statistics for 1 nM Raman sample (35 nm AuNP Dimer functionalized with 4-ATP dye, protected by SH-PEG). All the translocation experiments were performed in 100 nM KCl, 10 mM Tris-EDTA. The current-time trace is recorded with 10 us sampling rate and filtered using a low-pass Bessel filter with a cut-off frequency of 10 KHz. The dwell time of this sample is much longer than others in this work because the PEG modified molecules has high viscidity. Moreover, the charge of the particles is shield so they move slowly and some unpredictable rotation may happen when the electric field applied.

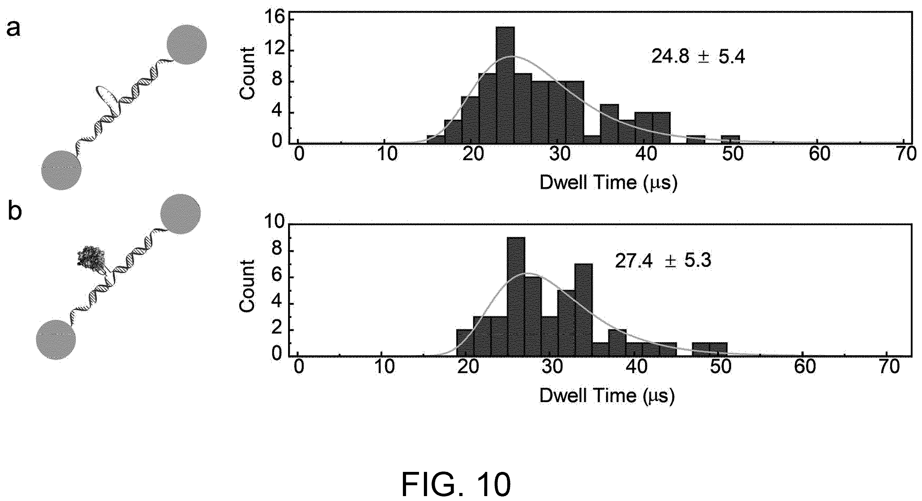

**Figure 10.** The translocation dwell time of AuNP dimer carrier with and without thrombin.
The dwell time of the translocation of **a** AuNP dimer thrombin carrier **b** AuNP dimer carrier with thrombin complex.

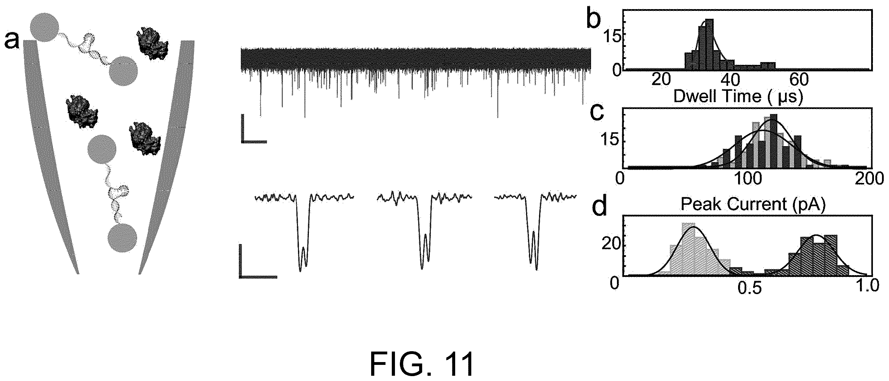

**Figure 11.** The control experiment of AuNP dimer thrombin carrier
The current-time trace (Scale bar: vertical 50 pA, horizontal 5 s), individual events (Scale bar: vertical 50 pA, horizontal 20 μs) and statistics for 1 nM AuNP dimer thrombin carrier incubated with 1 nM lysozyme. All the translocation experiments were performed in 50 mM KCl ,10 mM Tris-EDTA. The current-time trace is recorded with 1 μs sampling rate and filtered using a low-pass Bessel filter with a cut-off frequency of 100 KHz. There are no triple peak events but double peak events, corresponding to unbound thrombin carriers.

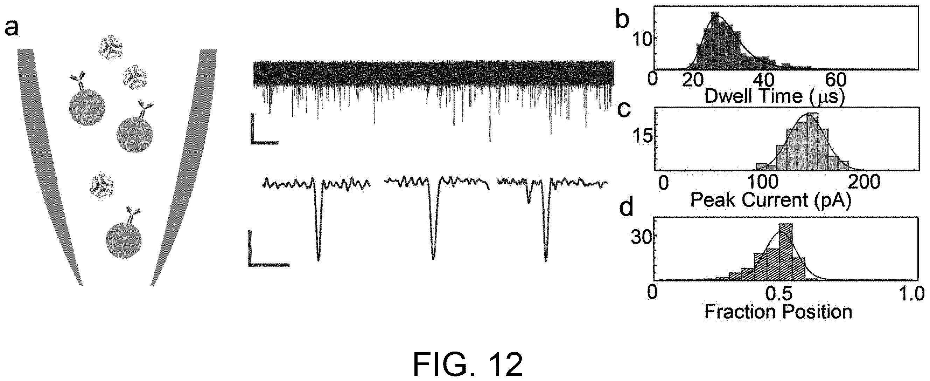

**Figure 12.** The control experiment of AuNP monomer PCT probe
The current-time trace (Scale bar: vertical 50 pA, horizontal 5 s), individual events (Scale bar: vertical 50 pA, horizontal 20 μs) and statistics for 1 nM AuNP monomer PCT probes incubated with 1 nM Insulin. All the translocation experiments were performed in 50 mM KCl ,10 mM Tris-EDTA. The current-time trace is recorded with 1 μs sampling rate and filtered using a low-pass Bessel filter with a cut-off frequency of 100 KHz. There are no double peak events but single peak events, corresponding to free PCT probes.

**Figure 13** Sensing analytes using antibody-functionalised NP probes and DNA carriers by biological nanopores.
**a** With the presence of the target protein, the aptamer-modified ssDNA carrier and the NP antibody probe will self-assemble to a ssDNA-protein-NP sandwich complex. **b** Schematic of working principle for nanopore sensing using DNA-NP self-assembly (top: working diagrams, bottom: typical signals). i. When the free ssDNA carrier translocates through the biological nanopore, the ionic current will be blocked by the DNA molecule and show a drop-down. ii. Since the protein cannot enter the nanopore, for ssDNA bound with target protein, the signal will show a subpeak due to the stuck or release of target protein. iii. With the form of ssDNA-protein-NP complex, the subpeak will be amplified due to the existence of NP. NP serves as a signal amplifier and effectively increases signal-to-noise ratio.

**Figure 14** Sensing miRNAs using DNA-functionalised NP probes and DNA carriers by biological nanopores.
**a** With the presence of the target miRNA, the ssDNA carrier with a complementary DNA (cDNA1) end to miRNA and the NP probe modified with the rest complementary DNA (cDNA2) will self-assemble to a ssDNA-miRNA-NP complex due to DNA-RNA hybridisation. **b** Schematic of working principle for nanopore sensing using DNA-NP self-assembly (top: working diagrams, bottom: typical signals). i. When free ssDNA translocates through the biological nanopore, the ionic current will be blocked by the DNA molecule and show a drop-down. ii. Since the hybridised dsDNA cannot enter the nanopore, for ssDNA bound with target miRNA, the signal will show a subpeak due to the stuck or release of target miRNA. iii. With the form of ssDNA-miRNA-NP complex, the subpeak will be amplified due to the existence of NP. NP serves as a signal amplifier and effectively increases signal-to-noise ratio.

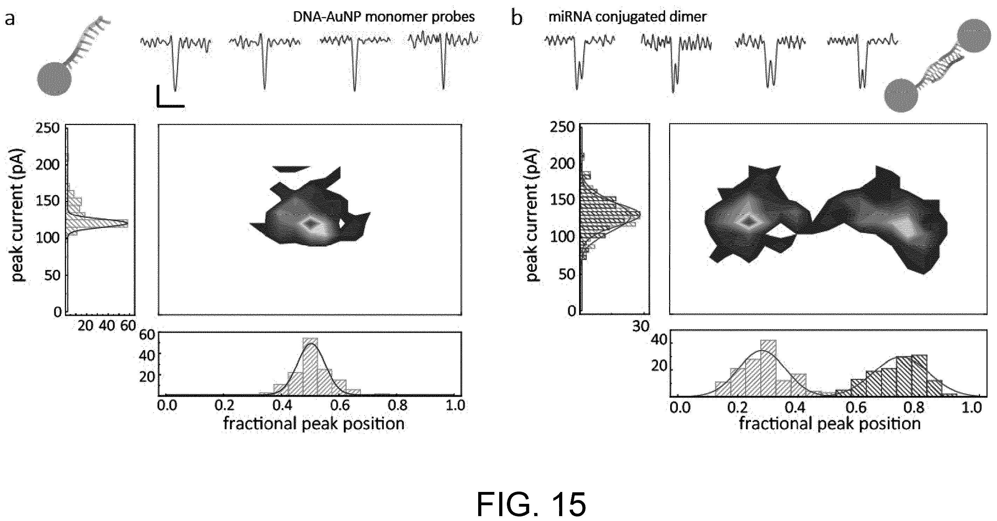

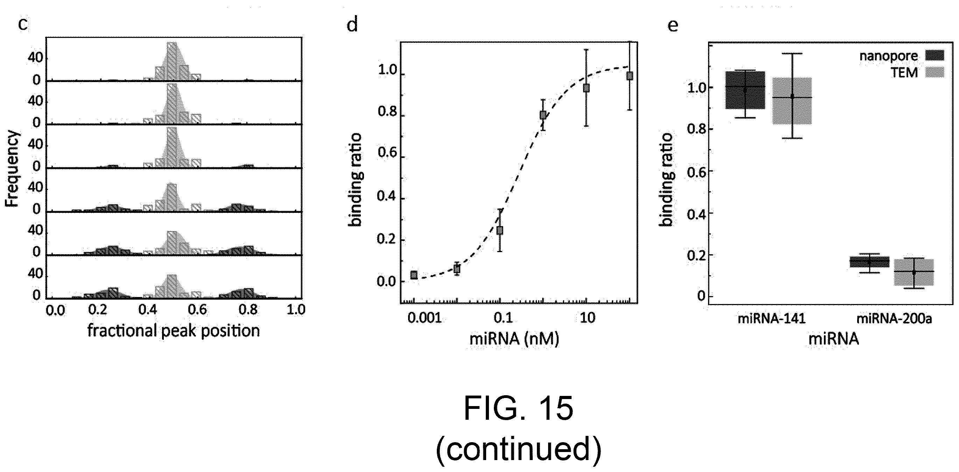

**Figure 15** Nanopore sensing of mRNA-141.
**(a)** AuNP monomer miRNA-141 molecular probes. Representative individual translocation events are shown (Scale bar: vertical 50 pA, horizontal 20 μs) along with associated statistics. **(b)** Conjugated dimers with miRNA141 linked between 2 NP monomers. All the translocation experiments were performed in 50 mM KCl, 10 mM Tris-EDTA and at a potential of -600 mV. **(c)** The normalised peak position of the monomer probe and conjugated dimer with different concentration of mRNA-141 (From top to bottom, 0 nM, 10 pM, 100 pM, 1 nM, 10 nM and 100 nM). **(d)** the binding curve of 2 nM AuNP monomer miRNA-141 probes incubating with the target miRNA ranging from 0 to 100 nM. (e) The comparison of the detection of miRNA-141 and miRNA-200a using AuNP monomer miRNA-141 probes. Error bars represent the standard deviation of three independent experimental repeats.

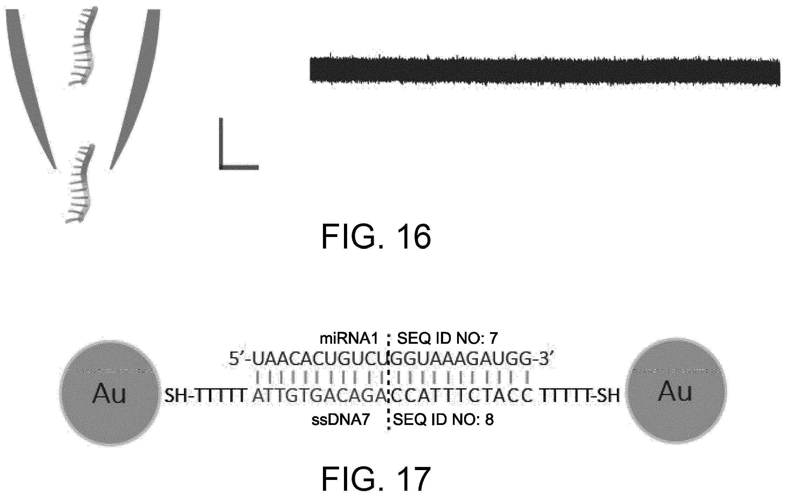

**Figure 16.** Sensing of miRNA without AuNP probes.
The current-time trace of miRNA-141 (10 nM) nanopore sensing (Scale bar: vertical 50 pA, horizontal 5 s). The translocation experiments were performed in 50 mM KCl, 10 mM Tris-EDTA, and at a potential of -600 mV. The current-time trace is recorded at a 1 MHz sampling rate and filtered using a low-pass Bessel filter with a cut-off frequency of 100 kHz. There are no observable translocations.

**Figure 17.** Schematic of miRNA-141 and AuNP monomer probes.
The miRNA-141 detection probes contain two types of ssDNA functionalized AuNP monomers. Each of them was modified by an 11 base recognition chain, which can hybridize with half of 22-base-long miRNA-141 (grey sequence in the figure). With the addition of the target, the monomer probes form a dimer. It is noteworthy that there are 5T close to the ssDNA recognition sequence to minimize the steric hindrance.

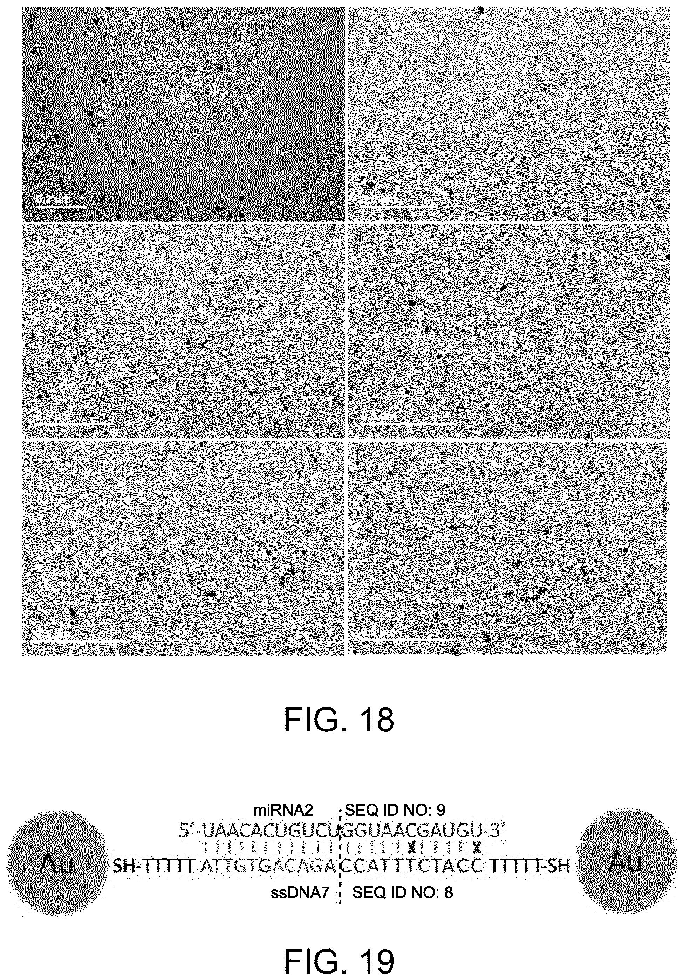

**Figure 18.** TEM images of AuNP monomer probes incubated with different levels of miRNA.
The TEM images (scale bars, 200 nm) of AuNP monomer probes incubated with (**a**) 0 nM (**b**) 10 pM (**c**) 100 pM (**d**) 1 nM (**e**) 10 nM (**f**) 100 nM miRNA-141.

**Figure 19.** The schematic of a miRNA-141 probe binding to miRNA-200a.
This was used as a control experiment and consisted of a two-base mismatch, which heavily decreases the affinity of hybridization.

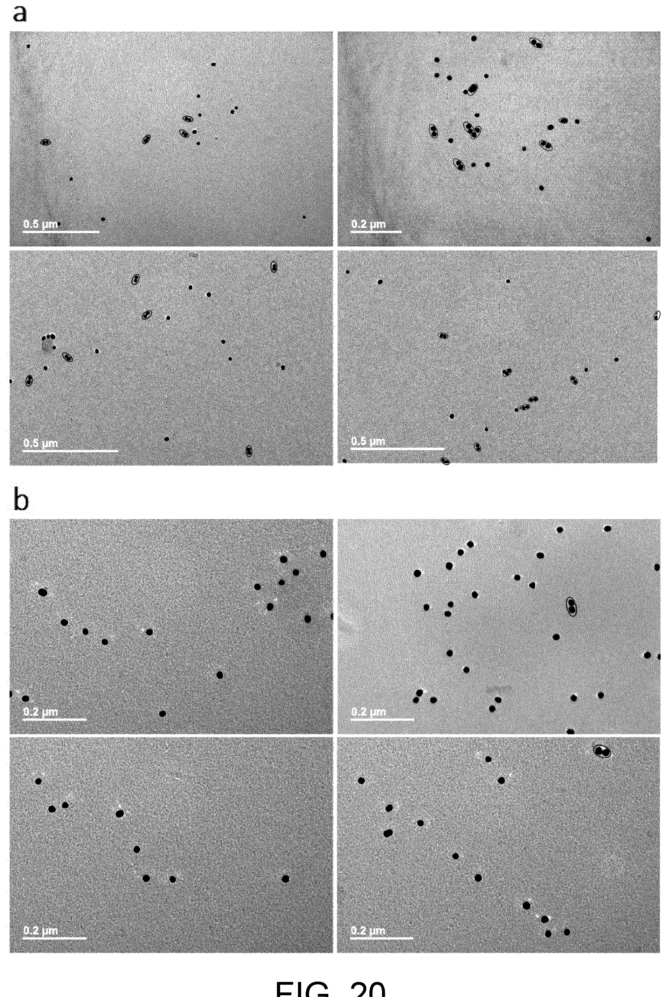

**Figure 20.** TEM images of the AuNP monomer miRNA-141 probe with the addition of miRNA-141 and miRNA-200a.
A large number of miRNA-141 probes (2 nM) dimerized to dumbbell molecules with the presence of (**a**) 100 nM miRNA-141, which is fully matched with the probe sequence, where only very few dimer molecules generated with the addition of (**b**) 100 nM miRNA-200a, which has two base mismatch with the AuNP probe.

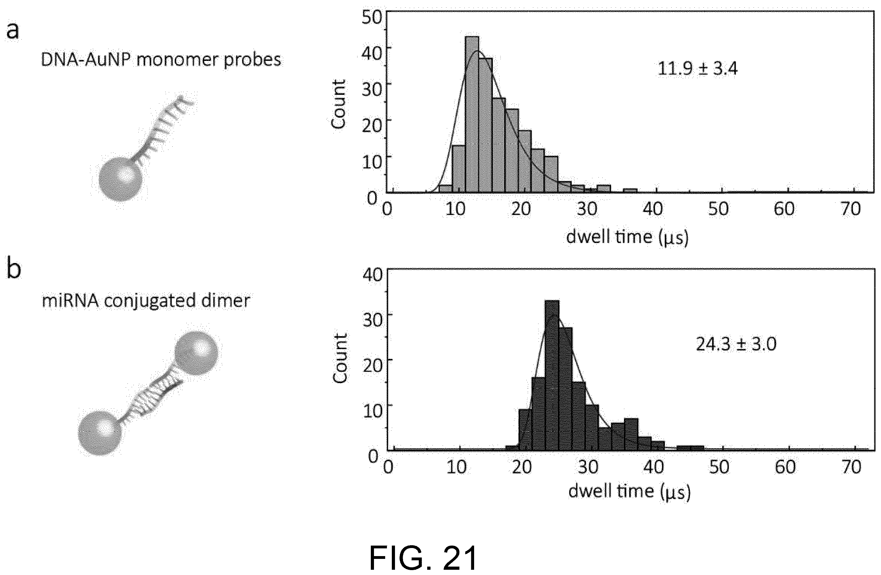

**Figure 21.** Dwell times for the translocation of (a) DNA-AuNP monomer probes (b) miRNA conjugated dimers.

## EP 4 158 340 B1

### Description of Sequences

The sequences referred to in the Figures are as follows:

| SEQ ID NO | Nucleic Acid | Figure | Sequence |
|---|---|---|---|
| SEQ ID NO: 1 | ssDNA1 | 7 | 5'-SH-TTTTTTTTTTTAGGTTCCCGATAAC-3' |
| SEQ ID NO: 2 | ssDNA2 | 7 | 5'-SH-TTTTTTTTTTTGTTATCGGGAACCTA-3' |
| SEQ ID NO: 3 | ssDNA3 | 7 | 5'-SH-TTTTTTTTTTGTTCAGTAACTAATTAACGTTGAAATGTCCGTAAGAACAGGAAATCACCAATAGACTCGACTTGGATACGCAGTGAATATGTCTATGCGTATCAATG-3' |
| SEQ ID NO: 4 | ssDNA4 | 7 | 5'-SH-TTTTTTTTTTTCATTGATACGCATAGACATATTCAC-3' |
| SEQ ID NO: 5 | ssDNA5 | 6 | 5'-SH-TTTTTTTTTTTGTCCAGTGGCTAATTAACGTTGAAATGTCCGTAAGAACAGGACATCACCAATAGCCTCGACTTGGATACGCAGTGCATACGTCTACGCGTAGGTTGGTGTGGTTGGATCGTCCGTCAATG-3' |
| SEQ ID NO: 6 | ssDNA6 | 6 | 5'-SH-TTTTTTTTTTTCATTGATACGCGTAGA-3' |
| SEQ ID NO: 7 | miRNA1 | 17 | 5'-UAACACUGUCUGGUAAAGAUGG-3' |
| SEQ ID NO: 8 | ssDNA7 | 17 and 19 | 5'-SH-TTTTTCCATCTTTACCAGACAGTGTTATTTTT-SH-3' |
| SEQ ID NO: 9 | miRNA2 | 19 | 5'-UAACACUGUCUGGUAACGAUGU-3' |

### Detailed Description

The ability to measure specific, selective biomarker molecules at single molecule level in physicians' surgeries and clinics has the potential to revolutionize disease diagnosis, monitoring, and therapy. Early and rapid diagnosis is an important factor to enhance the effectiveness of treatment. In many methods of early stage diagnosis, traditional biomarker detection methods like PCR and antigen-detecting ELISAs are widely used but limited due to many factors: 1) Low concentration of target biomarker molecules. People may be poor producers of an antibody or may have some interfering substance in their blood. The amount of antibody, consequently, may be too low to measure accurately or may go undetected. 2) Lack of single molecule data. This may cause the unspecific detection or recognise all isoforms of one same protein in a sample. 3) Other factors such as complex sample preparation and time consumption. Therefore, a fast, accurate and specific single molecule detection method is urgently needed.

Nanopores provide a label-free platform for sensing single biomolecules. Under applied potential, charged molecules will pass through the nanoscale pore and the resulting ionic current can be measured with standard electrophysiological techniques. Normally, long strand DNA molecules and large protein molecules can be distinguished easily by recognising the ionic current signal. However, this single molecule method is still challenging due to the low concentration in biological samples, fast translocation time of the analytes, poor analyte selectivity and low signal-to-noise ratio. Especially on the detection of protein molecules with small size and heterogeneous charge, such as lysozyme, thrombin and alpha synuclein, the single molecule signal becomes undetectable as the speed at which these molecules get transported through the nanopore is often too quick and hence hard to resolve. The present invention provides flexible, efficient and low-cost strategies to sense biomolecules of different sizes using a high resolution nanopore system.

The first aspect of the invention provides a series of molecular carriers and probes based on a nanoparticle dimer system, preferably a gold nanoparticle (AuNP) dimer system, which can deliver small analytes through a nanopore with improved signal-to-noise ratios.

The principle underlying the invention is increasing the effective size of the analyte using the nanoparticle dimer system. The signal of the dimer/analyte complex passing through a nanopore via voltage-driven translocation can be distinguished from the unbound dimer, allowing for detection even where the signal of the analyte cannot easily be detected.

The target analyte binds to dumbbell-shaped dimer carriers, with the corresponding aptamer located in the middle part, and then will be transported through a nanopore such as a fine-tuning nanopipette. Recorded by a high-bandwidth instrument, the high-res signal of nanoparticle monomers, nanoparticle dimers and nanoparticles with target protein can be differentiated by analysing the translocation events.

Moreover, this dimer system can be used to detect even smaller molecules which are highly likely undetectable in single molecule level. When the target protein is added into the solution, two nanoparticle monomers with different binding sites will link to each other and generate a nanoparticle-Antigen-nanoparticle dumbbell molecule because they will both bind to the target molecule. By detecting the ratio between the monomer and dimer, the high-res nanopore system not only can sense the antigens but also can quantify the trace amount concentration of them.

As described herein, the present invention is applicable to any nanoparticle which is suitable (in terms of its size and surface charge) for electrical detection when passed through a nanopore using voltage-driven translocation.

The sensing approaches of the present application are based on the differentiation of nanoparticles or nanoparticle-based conjugates. Herein, all nanoparticles with a suitable size and charge can be used in these methods. This includes nanoparticles formed of metals other than gold, alloys, polymers and silica. With different nanoparticles, the functionalised group on DNA may be changed to attach to it. Numerous nanoparticle (NP)/DNA conjugates, which can be used in the sensing system of the invention, have been reported (Samanta, A.; Medintz, I. L., Nanoparticles and DNA - a powerful and growing functional combination in bionanotechnology. Nanoscale 2016, 8 (17), 9037-9095) and examples of these are given in the table below.

| Nanoparticle | Constituents | DNA |
|---|---|---|
| AuNP | Au | FAM labeled T-rich DNA |
| AuNR | Au | Leukemia T cell targeting SH-DNA |
| Au/Ag hybrid | Au/Ag | Cytosine rich ssDNA |
| AgNP | Ag | SH-DNA |
| AgNC | Few Ag atoms | G-rich cocaine binding aptamer |
| MNP | $Fe_3O_4$ | Thrombin binding SH-DNA aptamer |
| PtNP | Pt | SH-DNA |
| PdNP | Pd | Thiol and amine functionalized oligos |
| QD | CdSe/ZnS | Dye-labeled photonic wire |
| SWCNT | Carbon | Ce6 conjugated thrombin binding aptamer |
| GO | Carbon | Short dsDNA with random sequence |
| Micelle | DNA + PPO | ssDNA covalently attached to PPO |
| Polyacrylamide-NP | Polyacrylamide | Dye-quencher labeled ATP aptamer |
| Viral NP | Bacteriophage MS2 capsid | Jurkat leukemia T cell specific DNA aptamer |
| Ferritin NPs | hFTN-H/eGFP or DsRed) | Amine modified PDGF specific aptamer |
| UCNP | $Yb^{3+}$ or $Tm^{3+}$ doped $NaYF_4$ | Amine-DNA with targeting sequence |
| Chalcogenide-NP | CuS | Amine modified targeting DNA |
| Alkaline earth | $Ca(H_2PO_4)_2,$ | eGFP encoding |
| metal NP | $CaHPO_4$ $Ca_3(PO_4)_2$ | plasmid DNA |
| **DNANP** | **DNA** | **>100 short oligomers,** |
| **Silane functionalised SiO2 NP** | **SiO2/Silane** | **Amine modified DNA** |

Abbreviations used in the table are as follows:

AuNP        Gold nanoparticle

| AuNR | Gold nanorod |
|------|--------------|
| AgNP | Silver nanoparticle |
| AgNC | Silver nanocluster |
| MNP | Magnetic nanoparticles |
| PtNP | Platinum nanoparticle |
| PdNP | Palladium nanoparticle |
| QD | Quantum dot |
| SWCNT | Single wall carbon nanotube |
| GO | Graphene oxide |
| UCNP | Upconversion nanoparticle |
| DNANP | Deoxyribonucleic acid nanoparticle |

In the present invention, the nanoparticle is typically a gold nanoparticle (AuNP). However, any of the other nanoparticles (NPs) described in the above table may alternatively be used.

There are many kinds of nanopore systems, including biological nanopore (e.g. alpha-hemolysin, MspA porin) and solid-state nanopore (e.g. Ion beam or electron beam drilled silicon nitrite membrane or graphene). In the Examples herein, nanopipettes were used among a variety of solid-state nanopore because of several advantages such as ease of fabrication, ease of set up (i.e., they can be tuned accurately) and low electrical noise. Nanopipettes may be manufactured by any suitable method available to the trained person. Quartz nanopipettes are particularly preferred as they are relatively easy to fabricate and do not introduce extra electrical noise or optical background.

Voltage-driven translocation through the nanopore may be achieved via any suitable means.

The ion currents of translocation are measured with the high-bandwidth amplifier such as a VC100 (Chimera Instruments). Typically, a grounded Faraday cage will be used to protect the nanopore system. For recording, a typical sampling rate is 1 MHz, and typically the data is filtered with 100 kHz low pass filter.

The inventors have designed a dumbbell-shaped nanoparticle dimer which is easy to fabricate and will cause specific signal shape when it passes through the nanopore. Ideally, a double peak event will be observed when a dimer molecule goes across the nanopore because the ionic signal will change while one nanoparticle is going into the pore and leaving. The dimer carrier will bind to the target protein molecule specifically by its aptamer branch. With the oligonucleotide sequences, aptamers are able to bind to their targets in a very specific way. Particularly, aptamers can be made to be applicable for almost any given target molecule, since aptamers can be collected through exponential enrichment process, in which ligands involved systematic evolution. Also, aptamers have other advantages such as low immunogenicity, small size, ease of modification and production and low toxicity. When a protein is transported by this dimer carrier, the changes of the double-peak event will give the information of this target molecule.

In one case, the nanoparticle monomer was based on AuNPs with 16-20 nm diameter with the surface functionalized by single stranded DNA (25-100 bases). However, nanoparticles of a variety of sizes may be used in the present invention. An AuNP dimer is 2 AuNP linked by a double stranded DNA. This dumbbell shape molecule can be self-assembled from two AuNP monomers with complementary ssDNA. The AuNP dimer protein carrier is a dimer with an aptamer branch in the middle. This dimer may be self-assembled from monomers with a ssDNA 1 (100 bases) and a ssDNA 2 (50 bases, DNA 2 is complementary with the end of DNA 1). The aptamer with DNA 3 (10-20 bases, DNA 3 is complementary with the unpaired part of DNA 1) then attached to the dimer. The resulting AuNPs dimer with a branch of aptamer can be the molecular carrier of a specific protein.

In an alternative embodiment, one nanoparticle (such as an AuNP) is functionalised with ssDNA with a part of aptamer sequence at the end while the other nanoparticle (such as an AuNP) is attached to ssDNA with the rest of the aptamer sequence; when the target protein is present, the two ssDNA parts of the aptamer will link together and form an aptamer which binds to the target protein, leading to the conjugation of the nanoparticle monomers. In this embodiment, the aptamer could be split in any proportion, as long as the parts link together to form a complete aptamer when the target protein is present.

It can therefore be seen that the aptamer may already be present in the DNA that is attached to one of the nanoparticles. Alternatively, the aptamer may be formed when the dimer forms, by hybridization of complementary single-stranded DNA that is bound to each of the nanoparticles.

By using the nanoparticle monomer probe, the system can be further extended to detect even smaller targets which are highly likely undetectable in single molecule level. Based on the result that the nanopore detection set up can distinguish nanoparticle monomers and dimers efficiently, the single molecule detection of small molecules can be achieved indirectly.. To calculate the ratio of the double-peak signal caused by dimer molecule translocation to the single peak signal caused by monomer translocation, we can know the accurate concentration of the targeting molecule.

The present invention will be further understood by reference to the following examples.

**Examples**

**Materials & Methods**

*The fabrication of nanopipettes*

The single barrel quartz capillaries (o.d., 1.0 mm, i.d., 0.7 mm, Intracell) were plasma cleaned (Harrick Plasma), and pulled with a laser-based P-2000 pipette puller (Sutter Instruments) using a two-line program (heat 800, filament 4, velocity 30, delay 170, and pull 80; heat 825, filament 3, velocity 20, delay 145, and pull 130) to produce nanopipettes with the nanopore diameters of approximately 34 nm at the tip as characterised by SEM imaging. It should be noted that the above pulling parameters are instrument specific and variations will exist from puller to puller.

*The fabrication of AuNP based nanostructures*

The effective length of a nanopipette is the portion of the electrolyte-filled pore over which the majority (for our calculations 75-80%) of its ionic resistance is focused. In addition, the voltage drop is greatest in this area, resulting a strongest electric field. For a cylindrical nanopore, the pore length (On the solid state nanopore always been considered as the thick of the membrane) equals to the effective length of the nanopore. Theoretically, the voltage drops linearly along the pore length in these cylindrical pores. However, the conical nanopore, which located on the tip of the nanopipette, results a nonlinear drop of the electric field and resistance along with the pore length because the pore radius changes along the distance to the end.

To detect the nanopipette effective length, the resistance distribution along the pore axis should be analysed (Figure. 5). The resistance with different position (Rx) of the nanopore can be estimated. The distance from the end, x, is ranging from 0 to L, which is a length that long enough and can be studied from the SEM images. D0 represents the diameter of the end of the nanopipette part and the DL represents the diameter of the area which L from the end. The diameter along with the distance, Dx, can be calculated. Then the Rx can be estimated.

Dx can be expressed as the following function of x:

$$D_x = \frac{x(D_L - D_0) + LD_0}{L} \tag{1}$$

In our calculation, the step of x is 1 nm.

The resistance Rx can be calculated as following function:

$$R_x = \frac{4L\rho}{\pi D_0 D_x} \tag{2}$$

where $\rho$ is the specific resistance of electrolyte which fills the pore. The $R_{tot}$ represents the total resistance.

*The fabrication of thrombin-binding aptamer (TBA) modified AuNP dimers*

The 17 nm AuNPs were concentrated 10-fold and resuspended in 0.5×TBE buffer to a final concentration of 10 nM. The prepared AuNPs were then mixed with the ssDNA5 (10 $\mu$M, dissolved in TE buffer), in a DNA: AuNP molar ratio of 5:1. NaCl solution (5 M) was added to the mixture to a final NaCl concentration of 50 mM, and left at 25 °C for 12 h. Finally, the mixture was centrifuged (three times) at 7000 rpm for 10 min to remove the excess DNA, and resuspended in TBE buffer. The process of AuNPs (17 nm) modified with the ssDNA6 were same as DNA5. 100 $\mu$L of AuNP-DNA5 and 100 $\mu$L of AuNP-DNA6 were mixed in TBE buffer containing 50 mM NaCl. The mixture was hybridised for 12 h at room temperature with gentle stirring. The mixture was centrifuged at 5000 rpm for 10 min to remove the single nanoparticles and collect the AuNPs dimer thrombin carrier. The sequence of DNA is shown in Figure 6.

*The fabrication of the monomer PCT probes*

2 mL AuNPs (20 ± 3 nm) were centrifuged for 10 min at 8000 rpm and then resuspended in 200 $\mu$L of 10 mM phosphate buffer (PB) solutions, which was adjusted to pH 9 with 0.1 M K2CO3. Next, 100 $\mu$L of the AuNPs were conjugated with anti-PCT mAb (10 $\mu$L, 100 $\mu$g/mL) and the other 100 $\mu$L AuNPs were modified with anti-PCT smAb (10 $\mu$L, 100 $\mu$g/mL), respectively. Then, the solutions were blocked by BSA (10 $\mu$L, 500 $\mu$g/mL) for 30 min after the incubation of 1h. Next, the functionalized AuNPs solutions were centrifuged for 10 min at 7500 rpm so that the final product can be collected as the monomer PCT probes.

## Nanostructures translocation experiment

The translocation experiments were performed from inside nanopipette to outside, where the AuNPs based nanostructures are loaded in the nanopipette which is the cis chamber as well as an Ag/AgCl working/patch electrode while an Ag/AgCl counter/reference electrode was placed in the blank buffer located in the bath as the trans chamber. The buffer used in the translocation experiments consisted of 50 mM KCl. 10 mM Tris-EDTA (pH=8) unless reported otherwise. For the binding assay of thrombin or PCT by using corresponding strategies, 1 nM thrombin carriers or PCT probes are incubated with target analytes at a different concentration at least 2 hrs. The buffer containing target analytes were filled inside the nanopipette along with the electrode. Then a voltage was applied by a high bandwidth amplifier VC100 (Chimera Instruments) between the electrodes on both sides of the nanopore, and the current-time trace can be recorded. The data was then resampled to 1 $\mu$s and refiltered to 100 kHz and was analysed by a customised Matlab App.

## Results

### Quantification of AuNP monomers, dimers, and trimers

To demonstrate the screening ability of the AuNP conjugates, we quantified the resolution of our platform. First, we confirmed the ability to differentiate between monomers and dimers linked with differing DNA lengths. AuNP monomers were fabricated by attaching a thiol-modified 25-mer ssDNA to the surface of 17 nm in diameter AuNPs (Fig. 2a). AuNP symmetrical dimers were fabricated by self-assembly of two AuNP monomers with one consisting of a 15 bases complementary sequence. To challenge the spatial resolution, we fabricated two kinds of AuNP symmetrical dimers: one with 35 bases linkers and the other with 115 bases linkers. Asymmetrical dimers we also designed consisting of 10 nm AuNP monomers and 20 nm AuNPs. Finally, trimers were also quantified and could be achieved by controlling the NP to DNA ratio.

### The fabrication of AuNP based nanostructures

### AuNP monomer

The synthesised AuNPs (17 nm) were concentrated 10-fold and resuspended in 0.5×TBE buffer to a final concentration of 10 nM. The prepared AuNPs were then modified with the ssDNA1 (10 $\mu$M, dissolved in TE buffer), in a DNA: AuNP molar ratio of 5:1. NaCl solution (5 M) was added to the mixture to a final NaCl concentration of 50 mM, and left at 25 °C for 12 h. Finally, the mixture was centrifuged (three times) at 7000 rpm for 10 min to remove the excess DNA and resuspended in TBE buffer. The schematic of AuNP monomer is shown in Figure 7a.

### AuNP symmetrical dimer (35 bases linker)

The process of AuNPs (17 nm) modified with the ssDNA2 were same as DNA1. 100 $\mu$L of AuNP-DNA1 and 100 $\mu$L of AuNP-DNA2 were mixed together in TBE buffer containing 50 mM NaCl. The mixture was hybridized for 12 h at room temperature with gentle stirring. The mixture was centrifuged at 5000 rpm for 10 min to remove the single nanoparticles and collect the AuNP symmetrical dimers with short linker. The schematic of AuNP symmetrical dimer (35 bases linker) is shown in Figure 7b.

### AuNP asymmetrical dimer

AuNPs were concentrated 10-fold and resuspended in 0.5×TBE buffer to a final concentration of 10 nM. The prepared 20 nm AuNPs were then modified with the ssDNA1 (10 $\mu$M, dissolved in TE buffer, mixture1) and 10 nm AuNPs were then modified with the ssDNA2 (10 $\mu$M, dissolved in TE buffer, mixture2), in a DNA: AuNP molar ratio of 5:1. NaCl solution (5 M) was added to the mixture to a final NaCl concentration of 50 mM, and left at 25 °C for 12 h. Finally, the mixture1 was centrifuged (three times) at 7000 rpm for 10 min, and the mixture2 was centrifuged (three times) at 8500 rpm for 10 min to remove the excess DNA and resuspended in TBE buffer. 100 $\mu$L of 20 nm AuNP-DNA1 and 100 $\mu$L of 10 nm AuNP-DNA2 were mixed together in TBE buffer containing 50 mM NaCl. The mixture was hybridized for 12 h at room temperature with gentle stirring. The mixture was centrifuged at 5000 rpm for 10 min to remove the single nanoparticles and collect the AuNP asymmetrical dimers. The schematic of AuNP asymmetrical dimer is shown in Figure 7c.

*AuNP trimer*

100 $\mu$L of 17 nm AuNP-DNA1 and 200 $\mu$L of 17 nm AuNP-DNA2 were mixed together in TBE buffer containing 50 mM NaCl. The mixture was hybridized for 12 h at room temperature with gentle stirring. The mixture was centrifuged at 4000 rpm for 10 min to remove the single nanoparticles and collect the AuNPs trimers. The schematic of AuNP trimer is shown in Figure 7d.

*AuNP symmetrical dimer (115 bases linker)*

The 17 nm AuNPs were concentrated 10-fold and resuspended in 0.5×TBE buffer to a final concentration of 10 nM. The prepared AuNPs were then modified with the ssDNA3 (10 $\mu$M, dissolved in TE buffer), in a DNA: AuNP molar ratio of 5:1. NaCl solution (5 M) was added to the mixture to a final NaCl concentration of 50 mM, and left at 25 °C for 12 h. Finally, the mixture was centrifuged (three times) at 7000 rpm for 10 min to remove the excess DNA, and resuspended in TBE buffer. The process of AuNPs (17 nm) modified with the ssDNA4 were same as DNA3. 100 $\mu$L of AuNP-DNA3 and 100 $\mu$L of AuNP-DNA4 were mixed together in TBE buffer containing 50 mM NaCl. The mixture was hybridized for 12 h at room temperature with gentle stirring. The mixture was centrifuged at 5000 rpm for 10 min to remove the single nanoparticles and collect the AuNP symmetrical dimers with long linker. The schematic of AuNP symmetrical dimer (115 bases linker) is shown in Figure 7e.

*4-ATP labelled AuNP symmetrical dimers (SERS sample)*

The 35 nm AuNPs were concentrated 10-fold and resuspended in 0.5×TBE buffer to a final concentration of 10 nM. The prepared AuNPs were then modified with the ssDNA1 (10 $\mu$M, dissolved in TE buffer), in a DNA: AuNP molar ratio of 5:1. NaCl solution (5 M) was added to the mixture to a final NaCl concentration of 50 mM, and left at 25 °C for 12 h. Finally, the mixture was centrifuged (three times) at 5000 rpm for 10 min to remove the excess DNA and resuspended in TBE buffer.

Before modified with the ssDNA2, 4-Aminothiophenol was added to the 35 nm AuNPs, with the final concentration of 10 $\mu$M. After 6 h, the AuNPs were centrifuged at 5000 rpm for 10 min to remove the unconnected 4-Aminothiophenol. Then, the above AuNPs were modified with ssDNA2. Finally, 100 $\mu$L of 35 nm AuNP-DNA1 and 100 $\mu$L of 35 nm AuNP-DNA2 were mixed together in TBE buffer containing 50 mM NaCl. The mixture was hybridized for 12 h at room temperature with gentle stirring. The mixture was centrifuged at 3000 rpm for 10 min to remove the single nanoparticles and collect the 4-ATP labelled AuNP symmetrical dimers. In order to increase the stability of the large AuNPs in high salt concentration, the SERS samples are further modified by PEG-SH. The schematic of 4-ATP labelled AuNP symmetrical dimers is shown in Figure 7f.

The geometry of the nanostructures was confirmed by transmission electron microscopy (TEM), Figure 2. To avoid the unpredictable orientation of the conjugated molecules with high viscosity during translocation, all AuNPs based samples were not protected by polyethylene glycol (PEG) unless otherwise stated. All NP conjugates were dispersed after fabrication and measured by UV-Vis after 24 hours stabilisation in the buffer 50 mM KCl, 10 mM Tris-EDTA and used in nanopore experiments. This ionic strength was chosen to minimise NP aggregation and at the same time maximise the signal to noise of the measurements.

Nanopore experiments were done using single-barrelled nanopipettes[39, 40], which can be fabricated by laser-assisted pulling of quartz capillaries. The nanopores were pulled with an average diameter slightly larger than the dimensions of the nanoparticles, with and average diameter of 34 $\pm$ 5 nm, as measured by scanning electron microscope (SEM). These dimensions closely matched the diameters estimated from nanopore conductance measurements () of 15.3 $\pm$ 2.4 nS in 100 mM KCl (n = 18). Using SEM imaging, the taper angle of the nanopipette tip was measured to be 16.9 $\pm$ 1.1° over the first 100 nm (n=18), which allowed us to estimate an effective sensing length between 25 to 50 nm, based on a 75-80% resistance drop at the nanopore. The analyte was filled inside the nanopipette where a patch electrode (AgCl) was placed, and a ground/reference electrode (AgCl) was placed in a bath, outside the pipette. By applying a negative potential, it was possible to transport the AuNPs from inside (cis) to outside (trans) of the nanopipette. A high bandwidth amplifier (Chimera VC100) was used with a 1 $\mu$s sampling rate and 100 kHz low-pass digital filter.

Figure 2 shows a comparison of translocation characteristics between the different conjugates. The simplest constructs, AuNP monomers, translocated relatively quickly in a single file translocation with a with a single peak dwell time distribution (mean dwell time of 8.3 $\pm$ 1.4 $\mu$s at an applied potential of -600 mV), Figure 2a. In comparison symmetric and asymmetric dimers with a 35 base DNA spacer, Fig. 2b-2c exhibited clear double file events. The current amplitudes of each subpeak are consistent with the size of the individual AuNPs within the conjugate. The observations are consistent with the model that the first peak appears due to the negatively charged AuNPs passing though the nanopore sensing area. This is followed by a decrease of the current due to the reduced charge on the linker. Finally, the 2nd AuNP results in a 2nd peak. The mean dwell time of the dimers in Fig. 2b is 20.8 $\pm$ 2.7 $\mu$s which is just over twice the monomer translocation time and is consistent with linear transport through the nanopore. Importantly, one could distinguish between the peaks with high spatial resolution, as the distance between the peaks was a mere 28 nm. Furthermore, it was possible to differentiate between spacers of different lengths, which correspond to distance of 28 and 51 nm, respectively.

When asymmetric AuNPs dimers were translocated out of the nanopipette (Fig. 2c), their asymmetric size was reflected in the shape of individual event in current time traces as well as the corresponding current peak distributions. The double peak current distributions were recorded with high peak currents of 155.51 ± 21.55 pA and 81.83 ± 13.47 pA, corresponding to translocations of 20 nm and 10 nm AuNP, respectively. Interestingly, nearly 95% of all translocation events of asymmetric dimers showed a preferential orientation with the larger NP being transported first, which was attributed to the larger NPs carrying higher surface charge. Although this was not the focus of the present manuscript, we also investigated the possibility of translocating and detecting NP trimers, Fig. 2d. Trimers could be detected with well-defined individual monomer signatures and it took 32.7 ± 5.0 $\mu$s for individual trimers to translocate through the nanopore, which is 3.94-folds (4.23-folds for spatial length) longer than the monomer dwell time and 1.57-folds (1.61- folds for spatial length) longer than the dimer dwell time, respectively.

To further characterise the translocations of these metallic nanoparticles, single particle surface-enhanced Raman scattering (SERS) was also performed on a modified gold coated (10 nm thickness) nanopipette. Due to the coupling and proximity between dimer and metal pipette surface, this results in a significant increase in Raman signal.[41, 42] To achieve single-particle SERS somewhat larger 35 nm AuNP symmetrical dimers we used due to the higher scattering cross-section. The AuNPs were functionalized with 4-Aminothiophenol (ATP) dye (Fig. 2e, Fig. 7). The dimer is further stabilized with PEG to ensure the particles do not aggregate at the 100 mM salt concentrations required to perform the translocations. A typical SERS spectrum of the NPs in bulk solution is shown and consists of expected peaks at 1138, 1387 and 1571 cm$^{-1}$ (Figure 8). This is comparable to the data obtained for single particle SERS as can be seen from the transients in Fig.2e. The integration time for the transients was 810 $\mu$s, and translocations were obtained at - 800 mV. In this example, the optical translocation times were 3.24 ms which is longer than the corresponding electrical events (0.79 ± 0.29 ms). This is due to the nanopore sensing region being much smaller than the diffraction limited laser spot size (ca. 1 $\mu$m). As a negative control, Raman spectra, which shows no Raman signal, were also acquired for the event when the reverse potential is applied to diffuse the dimer away from the nanopore. We envisage that this method can also be used to perform molecular assays and complements the electrical work shown in this manuscript.

*The design of AuNPs dimer thrombin carrier and the sensing of it with thrombin*

The first strategy for the biosensing is to utilise the dimer molecule as a carrier to drive and detect specific protein molecules. The AuNP dimer protein carrier, which is based on AuNP symmetrical dumbbell system with a DNA bridge, was engineered to contain a part of ssDNA overhang with the specific aptamer sequence. Due to the high affinity and selectivity of the aptamer and protein interaction, the dimer protein carriers with specific aptamer will only bind corresponding proteins in trace level, Fig. 3a.

In this case, human alpha-thrombin ($\alpha$-thrombin; M.W. 37.5 kDa; pl of 7.0-7.4), a multifunctional protease in the bloodstream, became our target due to its significant roles in various crucial physiological and pathological processes, such as blood coagulation, thrombosis and angiogenesis. It is essential to detect thrombin at a trace amount with high sensitivity. However, using plain solid-state nanopores to sense this biomarker with such small size and heterogeneous charge, is difficult to achieve. Therefore, AuNPs dimer thrombin carrier was designed to pave the way of thrombin detection at the single-molecule level.

The thrombin-binding aptamer (TBA), which binds to thrombin selectively and compactly ($K_d$ ~ 35-100 nM in solid phase assays[36]), is a 15-mer (5'-GGTTGGTGTGGTTGG-3' - SEQ ID NO: 10) ssDNA folding into stable intramolecular G-quadruplex in the presence of K$^+$.[43, 44] The TBA sequence was anchored on a protuberance of the dimer 115-mer DNA bridges as a binding site of thrombin located in the middle of the dumbbell molecules, Figure 6. Prior to nanopore measurements, the efficiency of the binding between thrombin and the corresponding carriers was confirmed by UV-vis. By adding 4 nM thrombin to 1 nM, AuNP dimer thrombin carrier dispersions with 50 mM KCl and 10 mM Tris-EDTA at pH 8, the AuNPs based molecules started oligomerisation due to the selective binding weakening the holistic charge of the molecule. As a control, the pristine AuNPs dimer molecules in the same ionic strength would not aggregate until the concentration of thrombin reached 100 nM, which masked the surface of the dimer molecules.

As a control, we first examine the translocation of TBA modified AuNP dimer, Fig. 3b. Comparing the translocation events of unmodified AuNPs dimer (Figure 10) and TBA modified AuNPs dimer (Fig. 3b) with same particle size and same linker length, the difference in dwell time, peak current, and fraction position are negligible. With the addition of 1 nM thrombin into 1 nM TBA modified AuNPs dimer, some triple peak events were observed when these complexes were driven by -600 mV potential, Fig. 3c. Unlike the triple peaks of the AuNPs trimers, the triple peaks events of the protein bound dimer revealed a distinct signature that the second peak (located on 0.51 of the normalised peak position) always smaller than the first and third peak (located on 0.22 and 0.86 of the normalised peak position, respectively). As discussed before, the peaks are generated when the nanopore conductance changed by the different part of the nanostructures. In the pH 8 environment, the thrombin and TBA part are negatively charged which is ascribable to the deprotonation of the amino acid, although it is not comparable with the charge of the AuNPs. Therefore, the small enhancement peak, with an 80% magnitude of the AuNPs peaks, occurred at the middle of the events, corresponding to the thrombin anchored DNA bridges. However, proved by the UV-Vis, the holistic charge of the molecular carrier is weakened after the addition of thrombin, which leading a 1.1-fold increase of the translocation dwell time, Figure 10.

By counting the percentage of the triple peak events of all triple peak and double peak events, the binding ratio can be calculated, Fig. 3d-e. As expected, the proportion of triple peak signature raised with the addition of thrombin. In this experiment, we varied the concentration of thrombin from 0 to 2 nM while the concentration of the dimer remained 1 nM. From the statistics of normalised peak position (Fig 3d), it is evident that there are no triple peak events without the addition of thrombin and then the middle peak showed stepwise with the increasing of the thrombin concentration. The subtle change of the concentration of thrombin can be monitored as approximate 50 pM change of the thrombin; the triple/double peak ratio will change 1%. Further, to validate the selectivity of the AuNP dimer thrombin carrier, 1 nM lysozyme was incubated with 1 nM thrombin carrier and subsequent nanopore detection of the mixture was performed, Figure 11. There is no sign of triple peak signature but the double peak events, which are corresponding to the unbounded dimer carrier.

*REFERENCE EXAMPLE - The design of AuNP monomer PCT probes and the detection of PCT in single molecule level*

The AuNP dimer protein carriers showed the ability to sense corresponding protein with high sensitivity and selectivity. However, the aptamers modified carriers, including most carriers reported previously, cannot sense smaller targets (<15kDa). What is worse, with the decreasing of the biomolecule size, the signatures become progressively hard to detect due to the lowering of the signal-to-noise ratio.

Herein, based on our previous work[45,46], a universal strategy of sensing small antigen molecules has been performed. In detail, half of AuNPs are modified by the corresponding antibody (mAb) of the target antigen while the rest are modified with complementary secondary antibody (smAb). With the presence of the antigen, the monomers will self-assemble to dimer with a 'sandwich' formation (Fig. 4a) and this changing can be recorded when the molecules pass through the nanopore.

In this case, we use the AuNP monomer antigen probes to detect procalcitonin (PCT; M.W. 12.8 kDa; pl of 6.5) which is a peptide precursor of the hormone calcitonin. Due to the PCT level variance between healthy and microbial infected individuals, it has become an important biomarker to improve bacterial infections identification and guide antibiotic therapy. A pair of AuNP monomer PCT probes were fabricated to sensing PCT in single-molecule level, which cannot be studied by the conventional translocation due to the extremely small size. To ensure the intramolecular nanostructure can be distinguished by the nanopore in this case, we increased the AuNP size to 20 nm. Therefore, with an antibody-antigen-antibody sandwich linker which is approximately 10 nm, the substructure of the dumbbell molecule (the centre of one AuNP to the other is around 30 nm) can be distinguished by nanopores with sub-30 nm effective length.

A comparison between the translocation of 2 nM AuNP monomers (50% are functionalized by PCT mAb, and 50% are functionalized by smAb) and the assembled dumbbell complex after adding 20 nM PCT, was shown in Fig. 4b-c. Without the presence of PCT, no double peak signal but a single peak signal was observed during the translocation of 2 nM AuNP monomers with 50 mM KCl, pH 8. With the addition of 20 nM PCT, about 15% of the single peaks transformed to clear double peak events. Each peak is the result of the translocating of AuNP whereas the trough is due to the translocation of the weak and uniform charged sandwich linker (PI of mAb, smAb is 6.6-7.2). As a negative control, no double peak signatures observed when PCT was replaced by other antigens, for example, insulin, Figure 12.

To validate the sandwich immunoassay mode can be used in clinical diagnosis level, the nanopore sensing studies of AuNP monomer probes with different concentration of PCT was performed. In this case, the concentration of the probes was kept as 2 nM while the concentration of PCT was ranging from 0 to 200 nM. As expected, the percentage of the single peak (located on 0.49 of the normalised peak position) decreased whereas the proportion of double peak signatures (located on 0.30 and 0.78 of the normalised peak position) raised with the increasing of PCT, Fig. 4d(i). The results were further confirmed by TEM (Fig. 4d(ii)), which provided visualised evidence that the proportion of dimer increased with more addition of PCT.

In bacterial infections, sepsis, severe sepsis and septic shock, PCT in plasma concentrations increases from 0.15 to more than 10 ng/ml. This increase often correlates with the severity of the disease and with mortality. At the same time, PCT has also been used to guide antibiotic therapy, for example, if PCT level < 0.1 ng/ml, antibiotic therapy is strongly discouraged; if PCT level > 1 ng/ml, antibiotic therapy is strongly encouraged. With high sensitivity, the PCT monomer probe is capable of sensing this important biomarker in this range (Fig. 4e inset).

*REFERENCE EXAMPLE - Molecular probes for single-molecule detection of miRNA*

miRNA are a class of short non-coding RNAs that function in RNA silencing and post-transcriptional gene regulation. Besides their participation in regulating normal physiological activities, specific miRNA types could act as oncogenes, tumor suppressors, or metastasis regulators, which are critical biomarkers for cancer. Conventional methods include Northern blotting, in situ hybridization, RT-qRCR, or microarrays. However, these methods require sample preparation or processing. In addition each technique has specific limitations such as low throughput and low sensitivity (for northern blotting), semi-quantitative (for in situ hybridization), time consuming, critical reaction condition (for RT-qPCR). Recent advances in nanopore technology offer the promise of addressing some of these drawbacks for detection of miRNA with high sensitivity and selectivity.[47] However, the signal of these short fragments (typically 18-23 bases) is hard to detect directly with solid-state nanopores due to the high-speed translocation and low signal-to-noise ratio, Figure 16. Here, we use AuNP dimer self-assembly to amplify this translocation signal, leading to very efficient miRNA detection at the single-molecule level.

In this study, we use AuNP molecular probes for the detection of miRNA-141. miRNA-141 is commonly dysregulated in malignant tumors such as those associated with prostate cancer and plays essential roles in tumor development and progression, becoming a powerful potential biomarker of prostate cancer.[48] Prostate cancer is the second most common cancer in men worldwide; however, disease outcome is difficult to predict in large part due to the lack of efficient diagnostic strategies. As such, miRNA-141 has the potential to become a useful biomarker.

The molecular probes consisted of two populations of ssDNA functionalized to AuNP monomers. Each of them was modified by an 11 base recognition chain, which can hybridize with half of the 22-base-long miRNA-141, Figure 17. With the addition of the target, the monomer probes self-assemble to form dimers and produce doublet signatures, Figure 15a-c. A binding assay was performed within the miRNA concentration range of 1 pM to 100 nM. As previously shown for PCT, the number of dimers, and hence doublets, increases with concentration, Figure 15d-e. Dimer formation is validated and compared with TEM, Figure 18, providing visual evidence of dimer formation due to the presence of miRNA-141. Typically, the concentration of miRNA-141 between fM and pM in unprocessed prostate cancer patient samples, and between pM to nM in extracted miRNA samples.

The specificity of the molecular probes was verified by detecting miRNA-200a, which is also in the miR-200 family and share seed sequences differing in only two nucleotides when compared with miRNA-141, Figure 19. The full recognition of miRNA-141 gives a significant binding result, whereas the control experiment, which is detecting the miRNA-200a, leads to a low value of the binding ratio, Figure 15e. The result is further confirmed by TEM, Figure 15e, Figure 20. Such high selective capability probably benefits from the dimerization mechanism. For example, for miRNA-141, the monomer probes can be linked to the dimer because the ssDNA is fully matching the target. In contrast, for the miRNA-200a, the two mismatch points happened on the same ssDNA of one monomer probe, leading to a very low binding affinity, which causes unsuccessful dimerization. This result shows that the AuNP monomer probe can detect the target with high specificity.

## Conclusion

Although nanoparticle-based superstructures have already been reported and some of them are detected by nanopore sensing[49], many of these tests are based on sensing the excluded volume rather than the substructures of these nanoparticle conjugates, leading to false positive in the nanoparticle based sensing applications. We demonstrate that it is possible to use a finely tuned nanopore testing platform incorporating with high-bandwidth instruments to depict the sub-structure of these molecules. We have shown this set-up can differentiate AuNP monomer, AuNPs symmetrical or asymmetrical dimer, and AuNPs trimer. By utilising the plasmonic effect of the dimer system, the single molecule SERS detection was also applied. Based on these validations of the detection resolution, two strategies of sensing biomolecules in a single molecule level are performed.

In summary, the first strategy (which is according to the invention) is to use an AuNPs dimer protein carrier, which is an AuNPs dumbbell system incorporating with an aptamer prominence on the middle of the DNA linker, to detect proteins. The second strategy exemplified, which is not according to the invention, is to utilise the feature of the self-assembling of AuNP monomer antigen probes to dimers with the addition of the specific antigens. Both strategies are fully flexible to detect a number of biomolecules with just changing the aptamer sequence or antibodies. The excellent selectivity and affinity of aptamer-protein or antibody-antigen provide the possibility to apply these strategies to diagnostics for detecting biomarkers in trace amount. Importantly, to different biomolecules, different strategy can be chosen. For example, to sense some large proteins, the aptamer modified carrier can be used because an evident signature in the middle can provide information such as the size and charge of the target. Otherwise, if the biomolecule is too small to generate the ripples on the electric signal, the other strategy, an indirect detection in single molecule level, can be applied. Both strategies are not only capable of validating the presence of the specific targets, but also can quantify the concentration of them in clinical diagnosis level.

References

1. Miles BN, Ivanov AP, Wilson KA, Dogan F, Japrung D, Edel JB. Single molecule sensing with solid-state nanopores: novel materials, methods, and applications. Chemical Society reviews 42, 15-28 (2013).

2. Meller A, Nivon L, Branton D. Voltage-driven DNA translocations through a nanopore. Physical review letters 86, 3435-3438 (2001).

3. Wanunu M. Nanopores: A journey towards DNA sequencing. Physics of life reviews 9, 125-158 (2012).

4. Ying YL, Zhang JJ, Gao R, Long YT. Nanopore-Based Sequencing and Detection of Nucleic Acids. Angewandte Chemie-International Edition 52, 13154-13161 (2013).

5. Wanunu M. Nanopores: A journey towards DNA sequencing. Physics of life reviews 9, 125-158 (2012).

6. Meller A, Branton D. Single molecule measurements of DNA transport through a nanopore. Electrophoresis 23, 2583-2591 (2002).

7. Li JL, Gershow M, Stein D, Brandin E, Golovchenko JA. DNA molecules and configurations in a solid-state nanopore microscope. Nature Materials 2, 611-615 (2003).

8. Fologea D, Gershow M, Ledden B, McNabb DS, Golovchenko JA, Li JL. Detecting single stranded DNA with a solid state nanopore. Nano letters 5, 1905-1909 (2005).

9. Wanunu M, Morrison W, Rabin Y, Grosbertg AY, Meller A. Electrostatic focusing of unlabelled DNA into nanoscale pores using a salt gradient. NatNanotechnol 5, 160-165 (2010).

10. Plesa C, Kowalczyk SW, Zinsmeester R, Grosberg AY, Rabin Y, Dekker C. Fast Translocation of Proteins through Solid State Nanopores. Nano letters 13, 658-663 (2013).

11. Li W, Bell NAW, Hernandez-Ainsa S, Thacker VV, Thackray AM, Bujdoso R, Keyser UF. Single Protein Molecule Detection by Glass Nanopores. ACS nano 7, 4129-4134 (2013).

12. Steinbock U, Krishnan S, Bulushev RD, Borgeaud S, Blokesch M, Feletti L, Radenovic A. Probing the size of proteins with glass nanopores. Nanoscale 6, 14380-14387 (2014).

13. Lan WJ, Holden DA, Zhang B, White HS. Nanoparticle Transport in Conical-Shaped Nanopores. Analytical Chemistry 83, 3840-3847 (2011).

14. Tsutsui M, Hongo S, He Y, Taniguchi M, Gemma N, Kawai T. Single-Nanoparticle Detection Using a Low-Aspect-Ratio Pore. ACS nano 6, 3499-3505 (2012).

15. Lan W-J, Holden DA, Liu J, White HS. Pressure-Driven Nanoparticle Transport across Glass Membranes Containing a Conical-Shaped Nanopore. Journal of Physical Chemistry C 115, 18445-18452 (2011).

16. Davenport M, Healy K, Pevarnik M, Teslich N, Cabrini S, Morrison AP, Siwy ZS, Letant SE. The Role of Pore Geometry in Single Nanoparticle Detection. ACS nano 6, 8366-8380 (2012).

17. Wang YX, Kececi K, Mirkin MV, Mani V, Sardesai N, Rusling JF. Resistive-pulse measurements with nanopipettes: detection of Au nanoparticles and nanoparticle-bound anti-peanut IgY. Chemical Science 4, 655-663 (2013).

18. Zahid OK, Wang F, Ruzicka JA, Taylor EW, Hall AR. Sequence-Specific Recognition of MiRNAs and Other Short Nucleic Acids with Solid-State Nanopores. Nano letters 16, 2033-2039 (2016).

19. Cai H, et al. Resistive-Pulse Measurements with Nanopipettes: Detection of Vascular Endothelial Growth Factor C (VEGF-C) Using Antibody-Decorated Nanoparticles. Analytical Chemistry 87, 6403-6410 (2015).

20. Heins EA, Siwy ZS, Baker LA, Martin CR. Detecting single porphyrin molecules in a conically shaped synthetic nanopore. Nano letters 5, 1824-1829 (2005).

21. Luan B, Stolovitzky G, Martyna G. Slowing and controlling the translocation of DNA in a solid-state nanopore. *Nanoscale* **4**, 1068-1077 (2012).

22. Keyser UF. Controlling molecular transport through nanopores. *Journal of the Royal Society Interface* **8**, 1369-1378 (2011).

23. Wei R, Gatterdam V, Wieneke R, Tampe R, Rant U. Stochastic sensing of proteins with receptor-modified solid-state nanopores. Nature nanotechnology 7, 257-263 (2012).

24. Ding S, Gao CL, Gu LQ. Capturing Single Molecules of Immunoglobulin and Ricin with an Aptamer-Encoded Glass Nanopore. Analytical Chemistry 81, 6649-6655 (2009).

25. Actis P, Rogers A, Nivala J, Vilozny B, Seger RA, Jejelowo O, Pourmand N. Reversible thrombin detection by aptamer functionalized STING sensors. Biosensors & Bioelectronics 26, 4503-4507 (2011).

26. Yusko EC, Johnson JM, Majd S, Prangkio P, Rollings RC, Li JL, Yang J, Mayer M. Controlling protein translocation through nanopores with bio-inspired fluid walls. Nature nanotechnology 6, 253-260 (2011).

27. Al Sulaiman D, Cadinu P, Ivanov AP, Edel JB, Ladame S. Chemically Modified Hydrogel-Filled Nanopores: A Tunable Platform for Single-Molecule Sensing. Nano letters 18, 6084-6093 (2018).

28. Xue L, Cadinu P, Nadappuram BP, Kang M, Ma Y, Korchev Y, Ivanov AP, Edel JB. Gated Single-Molecule Transport in Double-Barreled Nanopores. ACS applied materials & interfaces 10, 38621-38629 (2018).

29. Ren R, Zhang YJ, Nadappuram BP, Akpinar B, Klenerman D, Ivanov AP, Edel JB, Korchev Y. Nanopore extended field-effect transistor for selective single-molecule biosensing. Nature Communications 8, (2017).

30. Ai Y, Liu J, Zhang BK, Qian S. Field Effect Regulation of DNA Trans location through a Nanopore. Analytical Chemistry 82, 8217-8225 (2010).

31. Larkin J, Henley RY, Muthukumar M, Rosenstein JK, Wanunu M. High-Bandwidth Protein Analysis Using Solid-State Nanopores. Biophysical journal 106, 696-704 (2014).

32. Rosenstein JK, Wanunu M, Merchant CA, Drndic M, Shepard KL. Integrated nanopore sensing platform with sub-microsecond temporal resolution. Nature Methods 9, 487-U112 (2012).

33. Singer A, Wanunu M, Morrison W, Kuhn H, Frank-Kamenetskii M, Meller A. Nanopore Based Sequence Specific Detection of Duplex DNA for Genomic Profiling. Nano letters 10, 738-742 (2010).

34. Bell NAW, Keyser UF. Digitally encoded DNA nanostructures for multiplexed, single-molecule protein sensing with nanopores. Nature nanotechnology 11, 645-+ (2016).

35. Sze JYY, Ivanov AP, Cass AEG, Edel JB. Single molecule multiplexed nanopore protein screening in human serum using aptamer modified DNA carriers. Nature Communications 8, (2017).

35a. WO 2018/189530

36. Beamish E, Tabard-Cossa V, Godin M. Identifying Structure in Short DNA Scaffolds Using Solid-State Nanopores. Acs Sensors 2, 1814-1820 (2017).

37. Loh, AYY, Burgess CH, Tanase DA, FerrarG, McLachlan MA, Cass, AEG, Albrecht, T. Electric Single-Molecule Hybridization Detector for Short DNA Fragments. Analytical chemistry 23, 14063-14071 (2018).

38. Steinbock LJ, Otto O, Chimerel C, Gornall J, Keyser UF. Detecting DNA Folding with Nanocapillaries. Nano letters 10, 2493-2497 (2010).

39. Ying LM, White SS, Bruckbauer A, Meadows L, Korchev YE, Klenerman D. Frequency and voltage dependence of the dielectrophoretic trapping of short lengths of DNA and dCTP in a nanopipette. Biophysical journal 86, 1018-1027 (2004).

40. Karhanek M, Kemp JT, Pourmand N, Davis RW, Webb CD. Single DNA molecule detection using nanopipettes and nanoparticles. Nano letters 5, 403-407 (2005).

41. Nam JM, Oh JW, Lee H, Suh YD. Plasmonic Nanogap-Enhanced Raman Scattering with Nanoparticles. Accounts of Chemical Research 49, 2746-2755 (2016).

42. Cecchini MP, Wiener A, Turek VA, Chon H, Lee S, Ivanov AP, McComb DW, Choo J, Albrecht T, Maier SA, Edel JB. Rapid Ultrasensitive Single Particle Surface-Enhanced Raman Spectroscopy Using Metallic Nanopores. Nano letters 13, 4602-4609 (2013).

43. Deng B, Lin Y, Wang C, Li F, Wang Z, Zhang H, Li X-F, Le XC. Aptamer binding assays for proteins: The thrombin example-A review. Analytica Chimica Acta 837, 1-15 (2014).

44. Kelly JA, Feigon J, Yeates TO. Reconciliation of the X-ray and NMR structures of the thrombin-binding aptamer d(GGTTGGTGTGGTTGG). Journal of Molecular Biology 256, 417-422 (1996).

45. Wu XL, Xu LG, Liu LQ, Ma W, Yin HH, Kuang H, Wang LB, Xu CL, Kotov NA. Unexpected Chirality of Nanoparticle Dimers and Ultrasensitive Chiroplasmonic Bioanalysis. Journal of the American Chemical Society 135, 18629-18636 (2013).

46. Chen W, Bian A, Agarwal A, Liu LQ, Shen HB, Wang LB, Xu CL, Kotov NA. Nanoparticle Superstructures Made by Polymerase Chain Reaction: Collective Interactions of Nanoparticles and a New Principle for Chiral Materials. Nano letters 9, 2153-2159 (2009).

47. Dave VP, Ngo TA, Pernestig A-K, Tilevik D, Kant K, Nguyen T, et al. MicroRNA amplification and detection technologies: opportunities and challenges for point of care diagnostics. Laboratory Investigation. 99, 452-69 (2019).

48. Mitchell PS, Parkin RK, Kroh EM, Fritz BR, Wyman SK, Pogosova-Agadjanyan EL, et al. Circulating microRNAs as stable blood-based markers for cancer detection. Proceedings of the National Academy of Sciences of the United States of America. 105, 10513-8 (2008).

49. Ang YS, Yung L-YL. Rapid and Label-Free Single-Nucleotide Discrimination via an Integrative Nanoparticle-Nanopore Approach. ACS nano 6, 8815-8823 (2012).

## Claims

1. A method of detecting one or more analytes in a target sample, the method comprising:

   a. providing a nanoparticle dimer adapted to bind the analyte;
   b. causing the dimer to pass through a nanopore by voltage-driven translocation;
   c. observing changes in the translocation current; and
   d. comparing the translocation current profile of the target sample to the translocation current profile of a control sample;
   wherein a change in the translocation current profile of the target sample versus the control sample indicates the presence of the analyte in the target sample;
   wherein the dimer comprises two nanoparticles linked by one or more nucleic acids,
   wherein at least one of the nucleic acids includes an aptamer specific for the analyte; and
   wherein the analyte is a protein.

2. The method according to claim 1, wherein the nanoparticles are linked by partially complementary nucleic acids.

3. The method according to claim 1, wherein the dimer comprises two nanoparticles, each of which is attached to a nucleic acid, and each of the nucleic acids includes part of an aptamer specific for the analyte, such that in the presence of the analyte an aptamer is formed and thereby a dimer is formed.

4. The method of any preceding claim, wherein the nanoparticles in the dimer are of substantially the same diameter.

5. The method according to any one of claims 1 to 4, wherein the nanoparticles in the dimer are of different diameters.

6. The method of any preceding claim, wherein the nanopore is the tip of a nanopipette.

7. The method of any preceding claim, wherein the nanoparticle is a gold nanoparticle (AuNP).

8. The method of any preceding claim, wherein the target sample is a biological sample selected from blood, serum, lymph, sputum, urine, faeces, semen, sweat, tears, amniotic fluid, cerebrospinal fluid (CSF) and wound exudate.

**Patentansprüche**

1. Verfahren zum Nachweis eines oder mehrerer Analyten in einer Zielprobe, wobei das Verfahren Folgendes umfasst:

   a. Bereitstellen eines Nanopartikeldimers, das zur Bindung des Analyten ausgelegt ist;
   b. Bewirken des Durchgangs des Dimers durch eine Nanopore mittels spannungsgetriebener Translokation;
   c. Beobachten von Änderungen im Translokationsstrom; und
   d. Vergleichen des Translokationsstromprofils der Zielprobe mit dem Translokationsstromprofil einer Kontrollprobe;
   wobei eine Änderung des Translokationsstromprofils der Zielprobe gegenüber der Kontrollprobe das Vorliegen des Analyten in der Zielprobe anzeigt;
   wobei das Dimer zwei Nanopartikel umfasst, die durch eine oder mehrere Nukleinsäuren verknüpft sind, wobei wenigstens eine der Nukleinsäuren ein für den Analyten spezifisches Aptamer enthält; und
   wobei es sich bei dem Analyten um ein Protein handelt.

2. Verfahren nach Anspruch 1, wobei die Nanopartikel durch teilkomplementäre Nukleinsäuren verknüpft sind.

3. Verfahren nach Anspruch 1, wobei das Dimer zwei Nanopartikel umfasst, die jeweils an eine Nukleinsäure gebunden sind, und die Nukleinsäuren jeweils einen Teil eines für den Analyten spezifischen Aptamers enthalten, so dass in Gegenwart des Analyten ein Aptamer gebildet und dadurch ein Dimer gebildet wird.

4. Verfahren gemäß einem vorhergehenden Anspruch, wobei die Nanopartikel im Dimer im Wesentlichen den gleichen Durchmesser aufweisen.

5. Verfahren nach einem der Ansprüche 1 bis 4, wobei die Nanopartikel im Dimer unterschiedliche Durchmesser aufweisen.

6. Verfahren gemäß einem vorhergehenden Anspruch, wobei es sich bei der Nanopore um die Spitze einer Nanopipette handelt.

7. Verfahren gemäß einem vorhergehenden Anspruch, wobei es sich bei dem Nanopartikel um ein Gold-Nanopartikel (AuNP) handelt.

8. Verfahren gemäß einem vorhergehenden Anspruch, wobei es sich bei der Zielprobe um eine biologische Probe handelt, die aus Blut, Serum, Lymphe, Sputum, Urin, Kot, Samen, Schweiß, Tränen, Fruchtwasser, Liquor (Cerebrospinal Fluid, CSF) und Wundexsudat ausgewählt ist.

**Revendications**

1. Procédé de détection d'un ou plusieurs analytes dans un échantillon cible, le procédé comprenant :

   a. la fourniture d'un dimère de nanoparticule adapté pour se lier à l'analyte ;
   b. le passage du dimère à travers un nanopore par translocation commandée par tension ;

c. l'observation des changements dans le courant de translocation ; et

d. la comparaison du profil de courant de translocation de l'échantillon cible avec le profil de courant de translocation d'un échantillon témoin ;

dans lequel un changement dans le profil de courant de translocation de l'échantillon cible par rapport à l'échantillon témoin indique la présence de l'analyte dans l'échantillon cible ;

dans lequel le dimère comprend deux nanoparticules liées par un ou plusieurs acides nucléiques,

dans lequel au moins un des acides nucléiques comprend un aptamère spécifique de l'analyte ; et

dans lequel l'analyte est une protéine.

2. Procédé selon la revendication 1, dans lequel les nanoparticules sont liées par des acides nucléiques partiellement complémentaires.

3. Procédé selon la revendication 1, dans lequel le dimère comprend deux nanoparticules, dont chacune est fixée à un acide nucléique, et chacun des acides nucléiques comprend une partie d'un aptamère spécifique de l'analyte, de sorte qu'en présence de l'analyte, un aptamère est formé et ainsi un dimère est formé.

4. Procédé selon l'une quelconque des revendications précédentes, dans lequel les nanoparticules dans le dimère ont sensiblement le même diamètre.

5. Procédé selon l'une quelconque des revendications 1 à 4, dans lequel les nanoparticules dans le dimère ont des diamètres différents.

6. Procédé selon l'une quelconque des revendications précédentes, dans lequel le nanopore est la pointe d'une nanopipette.

7. Procédé selon l'une quelconque des revendications précédentes, dans lequel la nanoparticule est une nanoparticule d'or (AuNP).

8. Procédé selon l'une quelconque des revendications précédentes, dans lequel l'échantillon cible est un échantillon biologique choisi parmi le sang, le sérum, la lymphe, l'expectoration, l'urine, les fèces, le sperme, la sueur, les larmes, le liquide amniotique, le liquide céphalo-rachidien (LCR) et l'exsudat d'une plaie.

FIG. 1

FIG. 2

FIG. 2
(continued)

FIG. 3

FIG. 3
(continued)

FIG. 4

FIG. 4
(continued)

FIG. 5

ssDNA5 SEQ ID NO: 5
5'-SH-TTTTTTTTTGTCCAGTGGCTAATTAACGTTGAAATG TCCGTAAGAACAGGACATCACCAATAGCCTCGACTTGGAT ACGCAGTGCATACGTCTACGCGTAGGTTGGTGGTTGG ATCGTCCG TCAATG-3'

ssDNA6 SEQ ID NO: 6
5'-SH-TTTTTTTTTCATTGA TACGCGTAGA-3'

FIG. 6

a

NP ssDNA1
17 nm

ssDNA1 SEQ ID NO: 1
5'-SH-TTTTTTTTTTTAGGTTCCCGATAAC-3'

ssDNA2 SEQ ID NO: 2
5'-SH-TTTTTTTTTTTGTTATCGGGAACCTA-3'

b

NP ssDNA1 NP
ssDNA2
17 nm 17 nm

ssDNA3 SEQ ID NO: 3
5'-SH-TTTTTTTTTTTGTTCAGTAACTAATTAACGTTGAAAT
GTCCGTAAGAACAGGAAATCACCAATAGACTCGACTTG
GATACGCAGTGAATATGTCTATGCGTATCAATG-3'

c

NP ssDNA1 NP
ssDNA2
20 nm 10 nm

ssDNA4 SEQ ID NO: 4
5'-SH-TTTTTTTTTTTCATTGATACGCATAGACATATTCAC-3'

d

NP ssDNA1 NP ssDNA1 NP
ssDNA2 ssDNA2
17 nm 17 nm 17 nm

e

NP ssDNA3 NP
ssDNA4
17 nm 17 nm

f

4-Aminothiophenol

NP ssDNA1 NP S—⟨⟩—NH₂
ssDNA2
35 nm 35 nm

FIG. 7

FIG. 8

FIG. 9

FIG. 10

FIG. 11

FIG. 12

EP 4 158 340 B1

FIG. 13

**a**

ssDNA

target

NP probe

ssDNA/miRNA/NP conjugates

**b** (i)

(ii)

(iii)

(i)

(ii)

(iii)

current

time

ssDNA translocation

ssDNA translocation

miRNA stuck/release

ssDNA translocation

NP/miRNA /release

FIG. 14

FIG. 15

EP 4 158 340 B1

FIG. 15
(continued)

EP 4 158 340 B1

FIG. 16

miRNA1 ⦙ SEQ ID NO: 7
5′-UAACACUGUCUGGUAAAGAUGG-3′
||||||||||||⦙|||||||||||
SH-TTTTT ATTGTGACAGA⦙CCATTTCTACC TTTTT-SH
ssDNA7  ⦙SEQ ID NO: 8

FIG. 17

FIG. 18

miRNA2    SEQ ID NO: 9
5'-UAACACUGUCU GGUAACGAUGU-3'

SH-TTTTT ATTGTGACAGA CCATTTCTACC TTTTT-SH

ssDNA7   SEQ ID NO: 8

FIG. 19

a

b

FIG. 20

a

DNA-AuNP monomer probes

$11.9 \pm 3.4$

b

miRNA conjugated dimer

$24.3 \pm 3.0$

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2017091724 A1
- US 20190062814 A1
- WO 2007092434 A2
- WO 2018189530 A


### Non-patent literature cited in the description

- **LIN et al.** *Chem. Sci.*, 2017, vol. 8, 3905
- **Y. S. ANG et al.** *ACS Nano*, 2012, vol. 6 (10), 8815-8823
- **SALEH et al.** *PNAS*, 2003, vol. 100 (3), 820-824
- **SAMANTA, A.** ; **MEDINTZ, I. L.** Nanoparticles and DNA - a powerful and growing functional combination in bionanotechnology. *Nanoscale*, 2016, vol. 8 (17), 9037-9095
- **MILES BN** ; **IVANOV AP** ; **WILSON KA** ; **DOGAN F** ; **JAPRUNG D** ; **EDEL JB**. Single molecule sensing with solid-state nanopores: novel materials, methods, and applications. *Chemical Society reviews*, 2013, vol. 42, 15-28
- **MELLER A** ; **NIVON L** ; **BRANTON D**. Voltage-driven DNA translocations through a nanopore. *Physical review letters*, 2001, vol. 86, 3435-3438
- **WANUNU M**. Nanopores: A journey towards DNA sequencing. *Physics of life reviews*, 2012, vol. 9, 125-158
- **YING YL** ; **ZHANG JJ** ; **GAO R** ; **LONG YT**. Nanopore-Based Sequencing and Detection of Nucleic Acids. *Angewandte Chemie*, 2013, vol. 52, 13154-13161
- **MELLER A** ; **BRANTON D**. Single molecule measurements of DNA transport through a nanopore. *Electrophoresis*, 2002, vol. 23, 2583-2591
- **LI JL** ; **GERSHOW M** ; **STEIN D** ; **BRANDIN E** ; **GOLOVCHENKO JA**. DNA molecules and configurations in a solid-state nanopore microscope. *Nature Materials*, 2003, vol. 2, 611-615
- **FOLOGEA D** ; **GERSHOW M** ; **LEDDEN B** ; **MCNABB DS** ; **GOLOVCHENKO JA** ; **LI JL**. Detecting single stranded DNA with a solid state nanopore. *Nano letters*, 2005, vol. 5, 1905-1909
- **WANUNU M** ; **MORRISON W** ; **RABIN Y** ; **GROSBERG AY** ; **MELLER A**. Electrostatic focusing of unlabelled DNA into nanoscale pores using a salt gradient. *NatNanotechnol*, 2010, vol. 5, 160-165
- **PLESA C** ; **KOWALCZYK SW** ; **ZINSMEESTER R** ; **GROSBERG AY** ; **RABIN Y** ; **DEKKER C**. Fast Translocation of Proteins through Solid State Nanopores. *Nano letters*, 2013, vol. 13, 658-663
- **LI W** ; **BELL NAW** ; **HERNANDEZ-AINSA S** ; **THACKER VV** ; **THACKRAY AM** ; **BUJDOSO R** ; **KEYSER UF**. Single Protein Molecule Detection by Glass Nanopores. *ACS nano*, 2013, vol. 7, 4129-4134
- **STEINBOCK U** ; **KRISHNAN S** ; **BULUSHEV RD** ; **BORGEAUD S** ; **BLOKESCH M** ; **FELETTI L** ; **RADENOVIC A**. Probing the size of proteins with glass nanopores. *Nanoscale*, 2014, vol. 6, 14380-14387
- **LAN WJ** ; **HOLDEN DA** ; **ZHANG B** ; **WHITE HS**. Nanoparticle Transport in Conical-Shaped Nanopores. *Analytical Chemistry*, 2011, vol. 83, 3840-3847
- **TSUTSUI M** ; **HONGO S** ; **HE Y** ; **TANIGUCHI M** ; **GEMMA N** ; **KAWAI T**. Single-Nanoparticle Detection Using a Low-Aspect-Ratio Pore. *ACS nano*, 2012, vol. 6, 3499-3505
- **LAN W-J** ; **HOLDEN DA** ; **LIU J** ; **WHITE HS**. Pressure-Driven Nanoparticle Transport across Glass Membranes Containing a Conical-Shaped Nanopore. *Journal of Physical Chemistry C*, 2011, vol. 115, 18445-18452
- **DAVENPORT M** ; **HEALY K** ; **PEVARNIK M** ; **TESLICH N** ; **CABRINI S** ; **MORRISON AP** ; **SIWY ZS** ; **LETANT SE**. The Role of Pore Geometry in Single Nanoparticle Detection. *ACS nano*, 2012, vol. 6, 8366-8380
- **WANG YX** ; **KECECI K** ; **MIRKIN MV** ; **MANI V** ; **SARDESAI N** ; **RUSLING JF**. Resistive-pulse measurements with nanopipettes: detection of Au nanoparticles and nanoparticle-bound anti-peanut IgY. *Chemical Science*, 2013, vol. 4, 655-663
- **ZAHID OK** ; **WANG F** ; **RUZICKA JA** ; **TAYLOR EW** ; **HALL AR**. Sequence-Specific Recognition of MiRNAs and Other Short Nucleic Acids with Solid-State Nanopores. *Nano letters*, 2016, vol. 16, 2033-2039

- **CAI H et al.** Resistive-Pulse Measurements with Nanopipettes: Detection of Vascular Endothelial Growth Factor C (VEGF-C) Using Antibody-Decorated Nanoparticles. *Analytical Chemistry*, 2015, vol. 87, 6403-6410
- **HEINS EA** ; **SIWY ZS** ; **BAKER LA** ; **MARTIN CR**. Detecting single porphyrin molecules in a conically shaped synthetic nanopore. *Nano letters*, 2005, vol. 5, 1824-1829
- **WEI R** ; **GATTERDAM V** ; **WIENEKE R** ; **TAMPE R** ; **RANT U**. Stochastic sensing of proteins with receptor-modified solid-state nanopores. *Nature nanotechnology*, 2012, vol. 7, 257-263
- **DING S** ; **GAO CL** ; **GU LQ**. Capturing Single Molecules of Immunoglobulin and Ricin with an Aptamer-Encoded Glass Nanopore. *Analytical Chemistry*, 2009, vol. 81, 6649-6655
- **ACTIS P** ; **ROGERS A** ; **NIVALA J** ; **VILOZNY B** ; **SEGER RA** ; **JEJELOWO O** ; **POURMAND N**. Reversible thrombin detection by aptamer functionalized STING sensors. *Biosensors & Bioelectronics*, 2011, vol. 26, 4503-4507
- **YUSKO EC** ; **JOHNSON JM** ; **MAJD S** ; **PRANGKIO P** ; **ROLLINGS RC** ; **LI JL** ; **YANG J** ; **MAYER M**. Controlling protein translocation through nanopores with bio-inspired fluid walls. *Nature nanotechnology*, 2011, vol. 6, 253-260
- **AL SULAIMAN D** ; **CADINU P** ; **IVANOV AP** ; **EDEL JB** ; **LADAME S**. Chemically Modified Hydrogel-Filled Nanopores: A Tunable Platform for Single-Molecule Sensing. *Nano letters*, 2018, vol. 18, 6084-6093
- **XUE L** ; **CADINU P** ; **NADAPPURAM BP** ; **KANG M** ; **MA Y** ; **KORCHEV Y** ; **IVANOV AP** ; **EDEL JB**. Gated Single-Molecule Transport in Double-Barreled Nanopores. *ACS applied materials & interfaces*, 2018, vol. 10, 38621-38629
- **REN R** ; **ZHANG YJ** ; **NADAPPURAM BP** ; **AKPINAR B** ; **KLENERMAN D** ; **IVANOV AP** ; **EDEL JB** ; **KORCHEV Y**. Nanopore extended field-effect transistor for selective single-molecule biosensing. *Nature Communications*, 2017, vol. 8
- **AI Y** ; **LIU J** ; **ZHANG BK** ; **QIAN S**. Field Effect Regulation of DNA Trans location through a Nanopore. *Analytical Chemistry*, 2010, vol. 82, 8217-8225
- **LARKIN J** ; **HENLEY RY** ; **MUTHUKUMAR M** ; **ROSENSTEIN JK** ; **WANUNU M**. High-Bandwidth Protein Analysis Using Solid-State Nanopores. *Biophysical journal*, 2014, vol. 106, 696-704
- **ROSENSTEIN JK** ; **WANUNU M** ; **MERCHANT CA** ; **DRNDIC M** ; **SHEPARD KL**. Integrated nanopore sensing platform with sub-microsecond temporal resolution. *Nature Methods*, 2012, vol. 9, 487-U112
- **SINGER A** ; **WANUNU M** ; **MORRISON W** ; **KUHN H** ; **FRANK-KAMENETSKII M** ; **MELLER A**. Nanopore Based Sequence Specific Detection of Duplex DNA for Genomic Profiling. *Nano letters*, 2010, vol. 10, 738-742
- **BELL NAW** ; **KEYSER UF**. Digitally encoded DNA nanostructures for multiplexed, single-molecule protein sensing with nanopores. *Nature nanotechnology*, 2016, vol. 11, 645
- **SZE JYY** ; **IVANOV AP** ; **CASS AEG** ; **EDEL JB**. Single molecule multiplexed nanopore protein screening in human serum using aptamer modified DNA carriers. *Nature Communications*, 2017, vol. 8
- **BEAMISH E** ; **TABARD-COSSA V** ; **GODIN M**. Identifying Structure in Short DNA Scaffolds Using Solid-State Nanopores. *Acs Sensors*, 2017, vol. 2, 1814-1820
- **LOH, AYY** ; **BURGESS CH** ; **TANASE DA** ; **FERRARG** ; **MCLACHLAN MA** ; **CASS, AEG** ; **ALBRECHT, T**. Electric Single-Molecule Hybridization Detector for Short DNA Fragments. *Analytical chemistry*, 2018, vol. 23, 14063-14071
- **STEINBOCK LJ** ; **OTTO O** ; **CHIMEREL C** ; **GORNALL J** ; **KEYSER UF**. Detecting DNA Folding with Nanocapillaries. *Nano letters*, 2010, vol. 10, 2493-2497
- **YING LM** ; **WHITE SS** ; **BRUCKBAUER A** ; **MEADOWS L** ; **KORCHEV YE** ; **KLENERMAN D**. Frequency and voltage dependence of the dielectrophoretic trapping of short lengths of DNA and dCTP in a nanopipette. *Biophysical journal*, 2004, vol. 86, 1018-1027
- **KARHANEK M** ; **KEMP JT** ; **POURMAND N** ; **DAVIS RW** ; **WEBB CD**. Single DNA molecule detection using nanopipettes and nanoparticles. *Nano letters*, 2005, vol. 5, 403-407
- **NAM JM** ; **OH JW** ; **LEE H** ; **SUH YD**. Plasmonic Nanogap-Enhanced Raman Scattering with Nanoparticles. *Accounts of Chemical Research*, 2016, vol. 49, 2746-2755
- **CECCHINI MP** ; **WIENER A** ; **TUREK VA** ; **CHON H** ; **LEE S** ; **IVANOV AP** ; **MCCOMB DW** ; **CHOO J** ; **ALBRECHT T** ; **MAIER SA**. Rapid Ultrasensitive Single Particle Surface-Enhanced Raman Spectroscopy Using Metallic Nanopores. *Nano letters*, 2013, vol. 13, 4602-4609
- **DENG B** ; **LIN Y** ; **WANG C** ; **LI F** ; **WANG Z** ; **ZHANG H** ; **LI X-F** ; **LE XC**. Aptamer binding assays for proteins: The thrombin example-A review. *Analytica Chimica Acta*, 2014, vol. 837, 1-15
- **KELLY JA** ; **FEIGON J** ; **YEATES TO**. Reconciliation of the X-ray and NMR structures of the thrombin-binding aptamer d(GGTTGGTGTGGTTGG). *Journal of Molecular Biology*, 1996, vol. 256, 417-422

- **WU XL** ; **XU LG** ; **LIU LQ** ; **MA W** ; **YIN HH** ; **KUANG H** ; **WANG LB** ; **XU CL** ; **KOTOV NA**. Unexpected Chirality of Nanoparticle Dimers and Ultrasensitive Chiroplasmonic Bioanalysis. *Journal of the American Chemical Society*, 2013, vol. 135, 18629-18636
- **CHEN W** ; **BIAN A** ; **AGARWAL A** ; **LIU LQ** ; **SHEN HB** ; **WANG LB** ; **XU CL** ; **KOTOV NA**. Nanoparticle Superstructures Made by Polymerase Chain Reaction: Collective Interactions of Nanoparticles and a New Principle for Chiral Materials. *Nano letters*, 2009, vol. 9, 2153-2159
- **DAVE VP** ; **NGO TA** ; **PERNESTIG A-K** ; **TILEVIK D** ; **KANT K** ; **NGUYEN T et al.** MicroRNA amplification and detection technologies: opportunities and challenges for point of care diagnostics. *Laboratory Investigation*, 2019, vol. 99, 452-69
- **MITCHELL PS** ; **PARKIN RK** ; **KROH EM** ; **FRITZ BR** ; **WYMAN SK** ; **POGOSOVA-AGADJANYAN EL et al.** Circulating microRNAs as stable blood-based markers for cancer detection. *Proceedings of the National Academy of Sciences of the United States of America*, 2008, vol. 105, 10513-8
- **ANG YS** ; **YUNG L-YL**. Rapid and Label-Free Single-Nucleotide Discrimination via an Integrative Nanoparticle-Nanopore Approach. *ACS nano*, 2012, vol. 6, 8815-8823